(19) Europäisches Patentamt
European Patent Office
Office européen des brevets

(11) Veröffentlichungsnummer: **0 284 952**
**A2**

(12) # EUROPÄISCHE PATENTANMELDUNG

(21) Anmeldenummer: **88104541.3**

(22) Anmeldetag: **22.03.88**

(51) Int. Cl.⁴: **C07D 307/14 , C07D 307/52 , C07D 309/04 , C07D 333/20 , C07D 407/12 , C07D 409/12 , C07D 407/14 , C07D 409/14 , A01N 43/08 , A01N 43/10 , A01N 43/16**

(30) Priorität: **03.04.87 DE 3711345**

(43) Veröffentlichungstag der Anmeldung:
**05.10.88 Patentblatt 88/40**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Wollweber, Detlef, Dr.**
**Paul-Ehrlich-Strasse 17**
**D-5600 Wuppertal 1(DE)**
Erfinder: **Dutzmann, Stefan, Dr.**
**Leinenweberweg 33**
**D-4000 Düsseldorf 13(DE)**

(54) **Substituierte Propylamine.**

(57) Substituierte Propylamine der allgemeinen Formel (I)

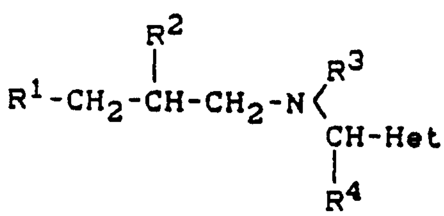

(I)

in welcher
$R^1$, $R^2$, $R^3$, $R^4$ und Het die in der Beschreibung gegebenen Bedeutungen haben und deren Verwendung zur Bekämpfung von Schädlingen.

Die neuen substituierten Propylamine der Formel (I) können nach bekannten Verfahren hergestellt werden, z.B. indem man geeignete Aldehyde mit geeigneten Aminen umsetzt oder in dem man geeignete Propylamine alkyliert bzw. hydriert.

EP 0 284 952 A2

## Substituierte Propylamine

Die Erfindung betrifft neue substituierte Propylamine, mehrere Verfahren zu ihrer Herstellung sowie ihr Verwendung als Schädlingsbekämpfungsmittel.

Es ist bereits bekannt, daß bestimmte substituierte Propylamine, wie beispielsweise das 1-(4-t-Butylphenyl)-2-methyl-3-(2,6-dimethylmorpholin-4-yl)-propan fungizide Eigenschaften besitzen (vgl. DE-OS 2 656 747).

Die Wirkung dieser vorgekannten Verbindungen ist jedoch insbesondere bei niedrigen Aufwandmengen und Konzentrationen nicht in allen Anwendungsbereichen völlig zufriedenstellend.

Es wurden neue substituierte Propylamine der allgemeinen Formel (I),

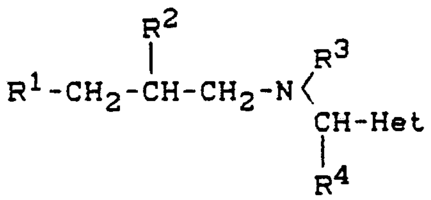

(I)

in welcher

$R^1$ für jeweils gegebenenfalls substituiertes Cycloalkyl, Cycloalkenyl, Tetrahydronaphthyl, Decahydronaphtyl, Phenyl, Naphthyl oder Thienyl steht,

$R^2$ für Alkyl steht,

$R^3$ für Wasserstoff, Alkyl, Alkenyl, Alkinyl, Hydroxyalkyl, Alkoxyalkyl, Dialkoxyalkyl, Hydroxyalkoxyalkyl, für Cyano, Formyl oder für jeweils gegebenenfalls substituiertes Cycloalkyl, Cycloalkenyl, Cycloalkylalkyl, Cycloalkenylalkyl, Aralkyl, Aryl, Furanylmethyl oder Tetrahydrofuranylmethyl steht,

$R^4$ für Wasserstoff oder Alkyl steht und

Het für einen gegebenenfalls substituierten Heterocyclus der Formel

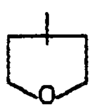 ; 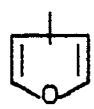 ; 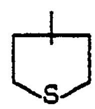 ; 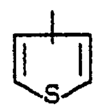 oder

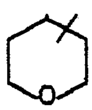 steht,

sowie deren pflanzenverträgliche Säureadditionssalze gefunden.

Weiterhin wurde gefunden, daß man die neuen substituierten Propylamine der allgemeinen Formel (I),

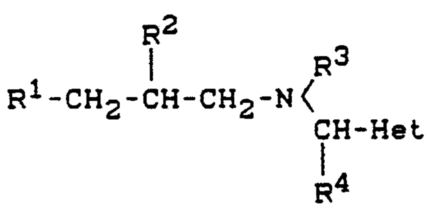

(I)

in welcher

$R^1$ für jeweils gegebenenfalls substituiertes Cycloalkyl, Cycloalkenyl, Tetrahydronaphthyl, Decahydronaphtyl, Phenyl, Naphthyl oder Thienyl steht,

$R^2$ für Alkyl steht,

$R^3$ für Wasserstoff, Alkyl, Alkenyl, Alkinyl, Hydroxyalkyl, Alkoxyalkyl, Dialkoxyalkyl, Hydroxyalkoxyalkyl, für Cyano, Formyl oder für jeweils gegebenenfalls substituiertes Cycloalkyl, Cycloalkenyl, Cycloalkylalkyl, Cycloalkenylalkyl, Aralkyl, Aryl, Furanylmethyl oder Tetrahydrofuranylmethyl steht,

$R^4$ für Wasserstoff oder Alkyl steht und
Het für einen gegebenenfalls substituierten Heterocyclus der Formel

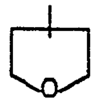 ; 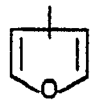 ; 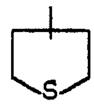 ; 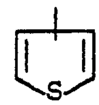 oder

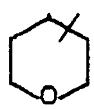

steht,
sowie deren pflanzenverträgliche Säureadditionssalze erhält, wenn man

(a) substituierte Aldehyde der Formel (II),

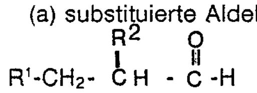 (II)

in welcher
$R^1$ und $R^2$ die oben angegebene Bedeutung haben,
mit Aminen der Formel (III),

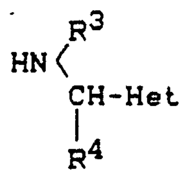 (III)

in welcher
$R^3$, $R^4$ und Het die oben angegebene Bedeutung haben,
in Gegenwart eines Reduktionsmittels sowie gegebenenfalls in Gegenwart eines Verdünnungsmittels und gegebenenfalls in Gegenwart eines Katalysators umsetzt oder wenn man

(b) die mit Hilfe des erfindungsgemäßen Verfahrens (a) erhältlichen substituierten Propylamine der Formel (Ia),

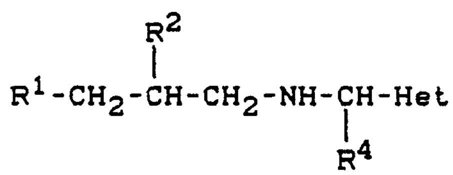 (Ia)

in welcher
$R^1$, $R^2$, $R^4$ und Het die oben angegebene Bedeutung haben,
mit Alkylierungsmitteln der Formel (IV),

$R^{3-1}$-E (IV)

in welcher
$R^{3-1}$ für Alkyl, Alkenyl, Alkinyl, Hydroxyalkyl, Alkoxyalkyl, Dialkoxyalkyl, Hydroxyalkoxyalkyl oder für jeweils gegebenenfalls substituiertes Cycloalkyl, Cycloalkenyl, Cycloalkylalkyl, Cycloalkenylalkyl, Aralkyl, Furanylmethyl oder Tetrahydrofuranylmethyl steht und
E für eine elektronenanziehende Abgangsgruppe steht,
gegebenenfalls in Gegenwart eines Verdünnungsmittels und gegebenenfalls in Gegenwart eines Reaktionshilfsmittels umsetzt, oder wenn man

(c) die mit Hilfe der erfindungsgemäßen Verfahren (a) oder (b) erhältlichen substituierten Propylamine der Formel (Ib),

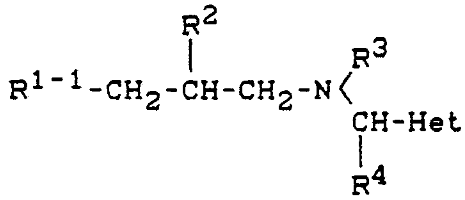

(Ib)

in welcher
$R^{1-1}$ für jeweils gegebenenfalls substituiertes Phenyl oder Naphthyl steht und
$R^2$, $R^3$, $R^4$ und Het die oben angegebene Bedeutung haben,
mit Wasserstoff in Gegenwart eines Katalysators sowie gegebenenfalls in Gegenwart eines Verdünnungsmittels hydriert und gegebenenfalls anschließend eine Säure addiert.

Schließlich wurde gefunden, daß die neuen substituierten Propylamine der allgemeinen Formel (I) eine gute Wirkung gegen Schädlinge, insbesondere gegen pilzliche Schädlinge besitzen.

Überraschenderweise zeigen die erfindungsgemäßen substituierten Propylamine der allgemeinen Formel (I) eine erheblich bessere fungizide Wirksamkeit als die aus dem Stand der Technik bekannten substituierten Propylamine, wie beispielsweise das 1-(4-t-Butylphenyl)-2-methyl-3-(2,6-dimethylmorpholin-4-yl)-propan, welches chemisch und wirkungsmäßig naheliegende Verbindungen sind.

Die erfindungsgemäßen substituierten Propylamine sind durch die Formel (I) allgemein definiert. Bevorzugt sind Verbindungen der Formel (I), bei welchen
$R^1$ für jeweils gegebenenfalls einfach bis mehrfach, gleich oder verschieden substituiertes Cycloalkyl oder Cycloalkenyl mit jeweils 3 bis 10 Kohlenstoffatomen steht, wobei als Substituenten infrage kommen: geradkettiges oder verzweigtes Alkyl mit 1 bis 8 Kohlenstoffatomen oder Cycloalkylalkyl mit 1 bis 8 Kohlenstoffatomen im geradkettigen oder verzweigten Alkylteil und 3 bis 7 Kohlenstoffatomen im Cycloalkylteil;
außerdem für jeweils gegebenenfalls einfach bis mehrfach, gleich oder verschieden substituiertes Tetrahydronaphthyl oder Decahydronaphthyl steht, wobei als Substituenten infrage kommen: jeweils geradkettiges oder verzweigtes Alkyl oder Alkoxy mit jeweils 1 bis 8 Kohlenstoffen;
weiterhin für jeweils gegebenenfalls einfach bis mehrfach, gleich oder verschieden substituiertes Phenyl oder Naphthyl steht, wobei als Substituenten jeweils infrage kommen: Halogen, geradkettiges oder verzweigtes Alkyl mit 1 bis 8 Kohlenstoffatomen, jeweils geradkettiges oder verzweigtes Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils gegebenenfalls durch geradkettiges oder verzweigtes Alkyl mit 1 bis 6 Kohlenstoffatomen einfach bis mehrfach, gleich oder verschieden substituiertes Cycloalkyl mit 3 bis 7 Kohlenstoffatomen, Aryl oder Aryloxy mit 6 bis 10 Kohlenstoffatomen sowie Arylalkyl mit 6 bis 10 Kohlenstoffatomen im Arylteil und 1 bis 6 Kohlenstoffatomen im geradkettigen oder verzweigten Alkylteil;
und schließlich für gegebenenfalls einfach bis mehrfach, gleich oder verschieden substituiertes Thienyl steht, wobei als Substituenten infrage kommen: Halogen, jeweils geradkettiges oder verzweigtes Alkyl oder Halogenalkyl mit jeweils 1 bis 8 Kohlenstoffatomen und gegebenenfalls 1 bis 9 gleichen oder verschiedenen Halogenatomen;
$R^2$ für geradkettiges oder verzweigtes Alkyl mit 1 bis 4 Kohlenstoffatomen steht,
$R^3$ für Wasserstoff, für geradkettiges oder verzweigtes Alkyl mit 1 bis 8 Kohlenstoffatomen, für jeweils geradkettiges oder verzweigtes Alkenyl oder Alkinyl mit jeweils 3 bis 8 Kohlenstoffatomen, für jeweils geradkettiges oder verzweigtes Alkoxyalkyl, Hydroxyalkoxyalkyl oder Dialkoxyalkyl mit jeweils 1 bis 4 Kohlenstoffatomen in den einzelnen Alkylteilen, für geradkettiges oder verzweigtes Hydroxyalkyl mit 2 bis 8 Kohlenstoffatomen, für Cyano, für Formyl, für jeweils gegebenenfalls einfach bis mehrfach, gleich oder verschieden durch geradkettiges oder verzweigtes Alkyl mit 1 bis 4 Kohlenstoffatomen substituiertes Cycloalkyl mit 3 bis 7 Kohlenstoffatomen, Cycloalkenyl mit 3 bis 7 Kohlenstoffatomen, Cycloalkylalkyl mit 3 bis 7 Kohlenstoffatomen im Cycloalkylteil und 1 bis 8 Kohlenstoffatomen im geradkettigen oder verzweigten Alkylteil, Cycloalkenylalkyl mit 3 bis 7 Kohlenstoffatomen im Cycloalkenylteil und 1 bis 8 Kohlenstoffatomen im geradkettigen oder verzweigten Alkylteil, Furanylmethyl oder Tetrahydrofuranylmethyl oder für jeweils gegebenenfalls einfach bis mehrfach, gleich oder verschieden substituiertes Phenylalkyl mit 1 bis 4 Kohlenstoffatomen im geradkettigen oder verzweigten Alkylteil oder Phenyl steht, wobei als Phenylsubstituenten jeweils infrage kommen: Halogen, jeweils geradkettiges oder verzweigtes Alkyl, Alkoxy oder Alkylthio mit jeweils 1 bis 4 Kohlenstoffatomen sowie jeweils geradkettiges oder verzweigtes Halogenalkyl, Halogenalkoxy oder Halogenalkylthio mit jeweils 1 bis 4 Kohlenstoffatomen und 1 bis 9 gleichen oder ver-

schiedenen Halogenatomen,
$R^4$ für Wasserstoff oder für geradkettiges oder verzweigtes Alkyl mit 1 bis 4 Kohlenstoffatomen steht und
Het für einen jeweils gegebenenfalls einfach bis mehrfach, gleich oder verschieden substituierten Heterocyclus der Formel

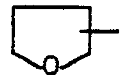 ; 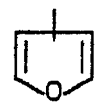 ;

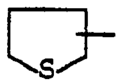 ; 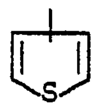 oder 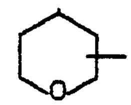

steht, wobei als Substituenten jeweils infrage kommen: jeweils geradkettiges oder verzweigtes Alkyl, Alkoxy, Alkylthio oder Alkoxycarbonyl mit jeweils 1 bis 4 Kohlenstoffatomen in den einzelnen Alkylteilen sowie jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen.

Besonders bevorzugt sind erfindungsgemäße Verbindungen der Formel (I), bei welchen
$R^1$ für jeweils gegebenenfalls ein-bis dreifach, gleich oder verschieden substituiertes Cyclohexyl oder Cyclohexenyl steht, wobei als Substituenten jeweils infrage kommen: Methyl, Ethyl, n-oder i-Propyl, n-, i-, s-oder t-Butyl, geradkettiges oder verzweigtes Pentyl, geradkettiges oder verzweigtes Hexyl, Cyclohexylmethyl, 1-Cyclohexylethyl, 1-Cyclohexylpropyl, 1-Cyclohexylbutyl, 2-Cyclohexyl-2-propyl sowie 2-Cyclohexyl-2-butyl;
außerdem für gegebenenfalls ein-bis dreifach, gleich oder verschieden substituiertes Tetrahydronaphthyl oder Decahydronaphthyl steht, wobei als Substituenten jeweils infrage kommen: Methyl, Ethyl, n-oder i-Propyl, n-, i-, s-oder t-Butyl, geradkettiges oder verzweigtes Pentyl, Methoxy, Ethoxy, n-oder i-Propoxy; weiterhin für jeweils gegebenenfalls ein-bis dreifach, gleich oder verschieden substituiertes Phenyl oder Naphthyl steht, wobei als Substituenten jeweils infrage kommen: Fluor, Chlor, Brom, Methyl, Ethyl, n-oder i-Propyl, n-, i-, s-oder t-Butyl, jeweils geradkettiges oder verzweigtes Pentyl, Hexyl, Heptyl oder Octyl, Methoxy, Ethoxy, n-oder i-Propoxy, n-, i-, s-oder t-Butoxy, Methylthio, Ethylthio, n-oder i-Propylthio, Trifluormethyl, Trifluormethoxy, Trifluormethylthio, Cyclohexyl, Phenyl, Phenoxy, Benzyl, 1-Phenylethyl, 1-Phenylpropyl, 2-Phenyl-2-propyl sowie 2-Phenyl-2-butyl, und schließlich für jeweils gegebenenfalls ein-bis dreifach, gleich oder verschieden durch Fluor, Chlor, Brom, Methyl, Ethyl, n-oder i-Propyl, n-, i-, s-oder t-Butyl oder Trifluormethyl substituiertes 2-Thienyl oder 3-Thienyl steht;
$R^2$ für Methyl, Ethyl, n-oder i-Propyl steht,
$R^3$ für Wasserstoff, Methyl, Ethyl, n-oder i-Propyl, n-, i-, s-oder t-Butyl, für jeweils geradkettiges oder verzweigtes Pentyl, Hexyl, Heptyl oder Octyl, für Allyl, Butenyl, Propargyl, Butinyl, Methoxyethyl, Methoxypropyl, Ethoxyethyl, Ethoxypropyl, Propoxypropyl, Dimethoxyethyl, Hydroxyethyl, Hydroxypropyl, Hydroxyethoxyethyl, für jeweils gegebenenfalls ein-bis dreifach, gleich oder verschieden substituiertes Cyclohexyl, Cyclohexenyl, Cyclopentyl, Cyclohexylmethyl, Cyclohexylethyl, Cyclohexenylmethyl, Cyclopentylmethyl, Furanylmethyl oder Tetrahydrofuranylmethyl steht, wobei als Substituenten jeweils infrage kommen: Methyl, Ethyl, n-oder i-Propyl, n-, i-, s-oder t-Butyl und außerdem für jeweils gegebenenfalls ein-bis dreifach gleich oder verschieden substituiertes Benzyl, Phenylethyl oder Phenyl steht, wobei als Phenylsubstituenten jeweils infrage kommen: Fluor, Chlor, Brom, Methyl, Ethyl, n-oder i-Propyl, Methoxy, Ethoxy, Methylthio, Trifluormethyl, Trifluormethoxy oder Trifluormethylthio,
$R^4$ für Wasserstoff, Methyl, Ethyl, n-oder i-Propyl steht und
Het für einen jeweils gegebenenfalls ein-bis dreifach, gleich oder verschieden substituierten Heterocyclus der Formel

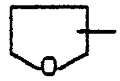 ; 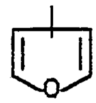 ; 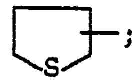 ;

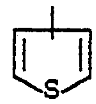 oder 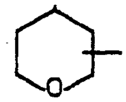

steht, wobei als Substi tuenten infrage kommen: Methyl, Ethyl, n-oder i-Propyl, n-, i-, s-oder t-Butyl, Methoxy, Ethoxy, n-oder i-Propoxy oder Methylthio.

Bevorzugte erfindungsgemäße Verbindungen sind auch Additionsprodukte aus Säuren und denjenigen substituierten Propylaminen der Formel (I), in denen die Substituenten $R^1$, $R^2$, $R^3$, $R^4$ und Het die Bedeutung haben, die bereits vorzugsweise für diese Substituenten genannt wurden.

Zu den Säuren die addiert werden können, gehören vorzugsweise Halogenwasserstoffsäuren, wie z.b. Chlorwasserstoffsäure und Bromwasserstoffsäure, insbesondere Chlorwasserstoffsäure, ferner Phosphorsäure, Salpetersäure, mono-, bi-und trifunktionelle Carbonsäuren und Hydroxycarbonsäuren, wie z.B. Ameisensäure, Essigsäure, Maleinsäure, Bernsteinsäure, Fumarsäure, Weinsäure, Zitronensäure, Salicylsäure, Sorbinsäure und Milchsäure, ferner Sulfonsäuren, wie p-Toluolsulfonsäure und 1,5-Naphthalindisulfonsäure sowie Saccharin.

Ganz besonders bevorzugt sind erfindungsgemäße Verbindungen der Formel (I), bei welchen

$R^1$ für jeweils gegebenenfalls ein-oder zweifach, gleich oder verschieden substituiertes Cyclohexyl oder Cyclohexenyl steht, wobei als Substituenten infrage kommen: i-Propyl, t-Butyl, neo-Pentyl, 1,1-Dimethylpropyl, 1,1,2-Trimethylpropyl, Cyclohexylmethyl oder 2-Cyclohexyl-2-propyl;

außerdem für gegebenenfalls ein-oder zweifach, gleich oder verschieden durch Methyl, Ethyl, Methoxy oder Ethoxy substituiertes Decahydronaphthyl steht;

weiterhin für jeweils gegebenenfalls ein-oder zweifach, gleich oder verschieden substituiertes Phenyl oder Naphthyl steht, wobei als Phenyl-Substituenten besonders bevorzugt sind: Fluor, Chlor, Brom, Isopropyl, t-Butyl, neo-Pentyl, 1,1-Dimethylpropyl, 1,1,2-Trimethylpropyl, Trifluormethyl, Trifluormethoxy, Cyclohexylmethyl, Cyclohexyl, Phenyl, Phenoxy, Benzyl, 1-Phenylethyl oder 2-Phenyl-2-propyl und wobei als Naphthyl-Substituenten besonders bevorzugt sind: Methyl, Ethyl, Methoxy oder Ethoxy und schließlich für jeweils gegebenenfalls durch Chlor, Methyl, i-Propyl oder t-Butyl substituiertes 2-Thienyl oder 3-Thienyl steht,

$R^2$ für Methyl steht,

$R^3$ für Wasserstoff, Methyl, Ethyl, n-oder i-Propyl, n-, i-oder s-Butyl, n-Pentyl, n-Hexyl, n-Octyl, Allyl, Propargyl, Methoxyethyl, Methoxypropyl, Ethoxyethyl oder Ethoxypropyl steht,

$R^4$ für Wasserstoff oder Methyl steht und

Het für einen gegebenenfalls durch Methyl substituierten Heterocyclus der Formel

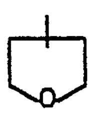 ; 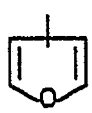 ; oder 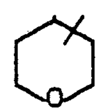

steht.

Halogen steht auch in den Zusammensetzungen wie Halogenalkyl für Fluor, Chlor, Brom und Jod, insbesondere für für Fluor, Chlor und Brom, wenn an anderer Stelle nicht anders angegeben. Bevorzugt sind auch die Verbindungen, in denen Het in der Formel (I) in 2-oder 3-Position verknüpft sind.

Besonders bevorzugt sind außerdem Säureadditionsverbindungen aus Verbindungen der Formel (I) mit Chlorwasserstoff, Ameisensäure, Essigsäure, Methansulfonsäure, p-Toluolsulfonsäure, 1,6-Naphthalindisulfonsäure und Saccharin.

Im einzelnen seien außer den bei den Herstellungsbeispielen genannten Verbindungen die folgenden substituierten Propylamine der allgemeinen Formel (I) genannt:

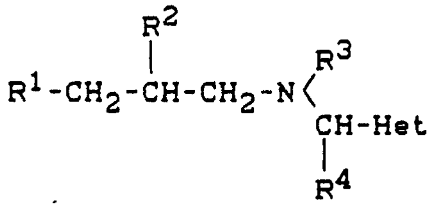 (I)

| $R^1$ | $R^2$ | $R^3$ | $R^4$ | Het |
|---|---|---|---|---|
| $(CH_3)_3C$ 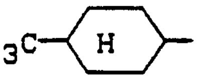 | $CH_3$ | $-(CH_2)_2-CH_3$ | H | 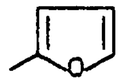 |
| $(CH_3)_3C$ 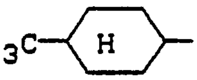 | $CH_3$ | $-(CH_2)_2-CH_3$ | H | 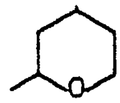 |
| $(CH_3)_3C$ 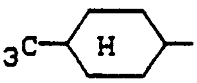 | $CH_3$ | $-(CH_2)_2-CH_3$ | H | 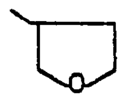 |

| $R^1$ | $R^2$ | $R^3$ | $R^4$ | Het |
|---|---|---|---|---|
| 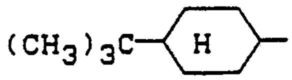 | $CH_3$ | $-(CH_2)_2-CH_3$ | H | 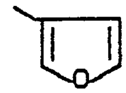 |
| 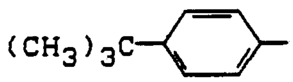 | $CH_3$ | $-(CH_2)_2-CH_3$ | H | 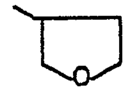 |
| 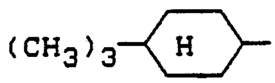 | $CH_3$ | $-(CH_2)_2-CH_3$ | H | 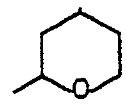 |
| 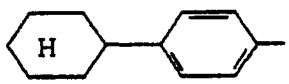 | $CH_3$ | $-CH_2-CH(CH_3)_2$ | H | 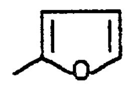 |
| 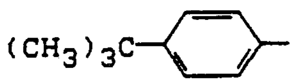 | $CH_3$ | $-(CH_2)_2-CH_3$ | H | 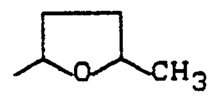 |
| 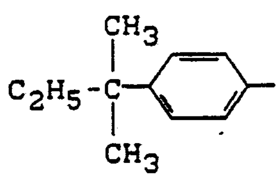 | $CH_3$ | $-(CH_2)_2-CH_3$ | H | 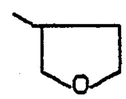 |
| 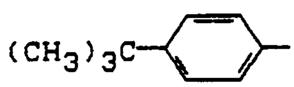 | $CH_3$ | $-(CH_2)_2-CH_3$ | $CH_3$ | 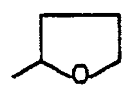 |
| 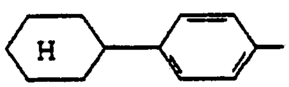 | $CH_3$ | $-(CH_2)_2-CH_3$ | $CH_3$ | 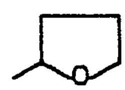 |
| 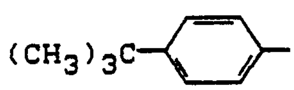 | $-CH(CH_3)_2$ | 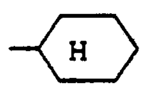 | $C_2H_5$ | 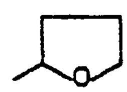 |

| $R^1$ | $R^2$ | $R^3$ | $R^4$ | Het |
|---|---|---|---|---|
| 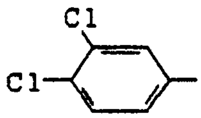 | $-(CH_2)_3-CH_3$ | $C_2H_5$ | $CH_3$ | 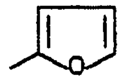 |
| 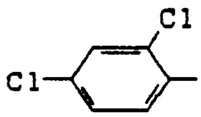 | $C_2H_5$ | $C_2H_5$ | $C_2H_5$ | 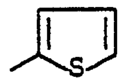 |
| 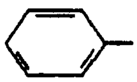 | $CH_3$ | $-(CH_2)_2-CH_3$ | H | 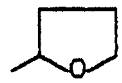 |
| 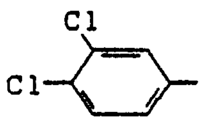 | $CH_3$ | $-(CH_2)_2-CH_3$ | H | 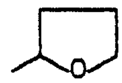 |
| 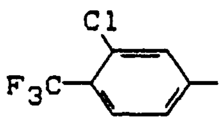 | $CH_3$ | $-(CH_2)_2-CH_3$ | H | 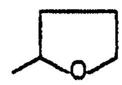 |
| 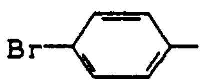 | $CH_3$ | $-(CH_2)_2-CH_3$ | H | 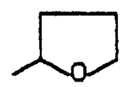 |
| 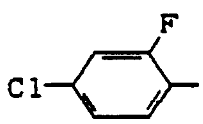 | $CH_3$ | $-(CH_2)_2-CH_3$ | H | 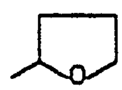 |
| 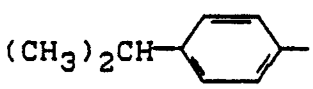 | $CH_3$ | $-(CH_2)_2-CH_3$ | H | 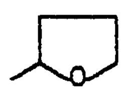 |

| $R^1$ | $R^2$ | $R^3$ | $R^4$ | Het |
|---|---|---|---|---|
| 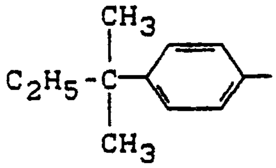 | $CH_3$ | $-(CH_2)_2-CH_3$ | H | 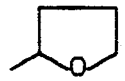 |
| 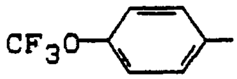 | $CH_3$ | $-(CH_2)_2-CH_3$ | H | 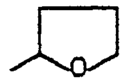 |
| 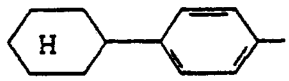 | $CH_3$ | $-(CH_2)_2-CH_3$ | H | 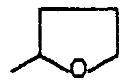 |
| 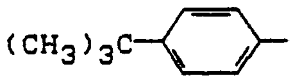 | $CH_3$ | H | H | 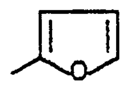 |
| 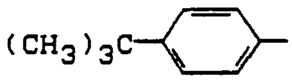 | $CH_3$ | H | H | 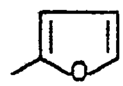 |
| 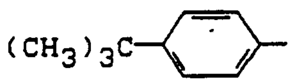 | $CH_3$ | $CH_3$ | H | 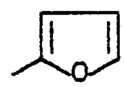 |
| 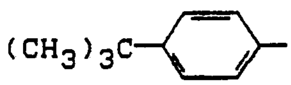 | $CH_3$ | $C_2H_5$ | H | 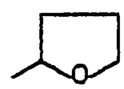 |
| 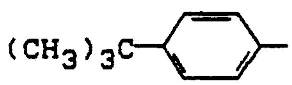 | $CH_3$ | $-(CH_2)_3-CH_3$ | H | 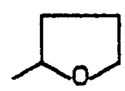 |
| 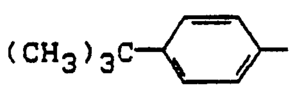 | $CH_3$ | $-CH(CH_3)_2$ | H | 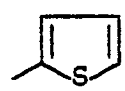 |

| $R^1$ | $R^2$ | $R^3$ | $R^4$ | Het |
|---|---|---|---|---|
| 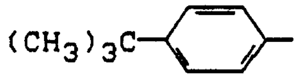 | $CH_3$ | 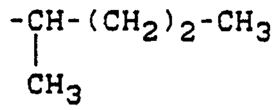 | H | 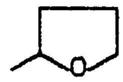 |
| 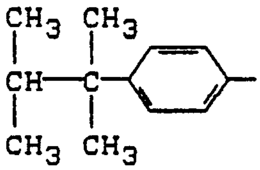 | $CH_3$ | $-CH_2-CH(CH_3)_2$ | H | 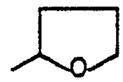 |
| 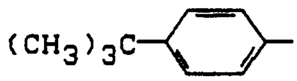 | $CH_3$ | $-C(CH_3)_3$ | H | 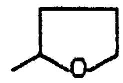 |
| 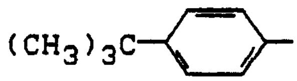 | $CH_3$ | $-(CH_2)_7-CH_3$ | H | 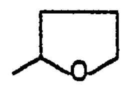 |
| 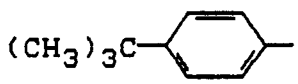 | $CH_3$ | 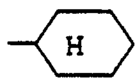 | H | 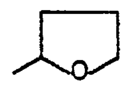 |
| 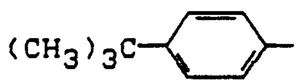 | $CH_3$ | 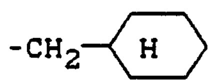 | H | 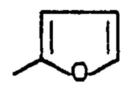 |
| 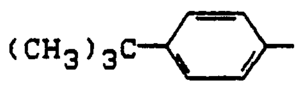 | $CH_3$ | 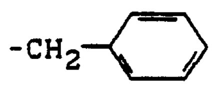 | H | 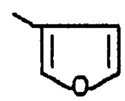 |
| 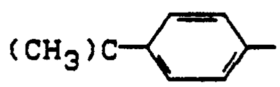 | $CH_3$ | 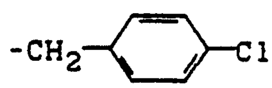 | H | 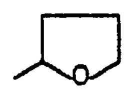 |

| $R^1$ | $R^2$ | $R^3$ | $R^4$ | Het |
|---|---|---|---|---|
| 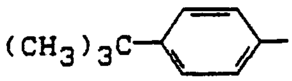 | $CH_3$ | 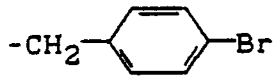 | H | 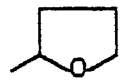 |
| 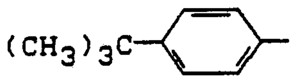 | $CH_3$ | 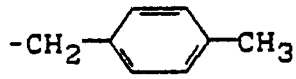 | H | 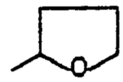 |
| 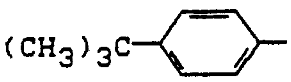 | $CH_3$ | 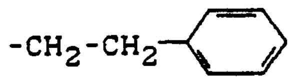 | H | 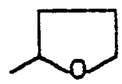 |
| 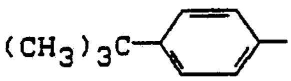 | $CH_3$ | 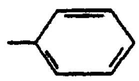 | H | 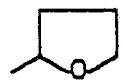 |
| 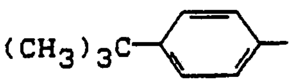 | $CH_3$ | $-CH_2-CH_2-OCH_3$ | H | 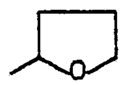 |
| 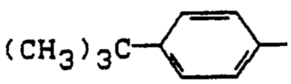 | $CH_3$ | $-(CH_2)_3-OCH_3$ | H | 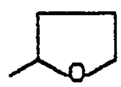 |
| 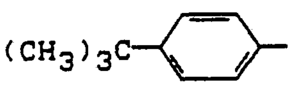 | $CH_3$ | $-(CH_2)_3-OC_2H_5$ | H | 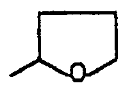 |
| 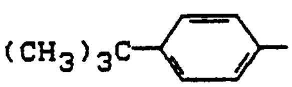 | $CH_3$ | 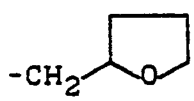 | H | 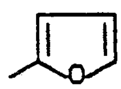 |
| 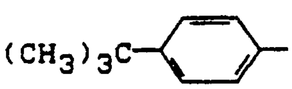 | $CH_3$ | 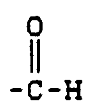 | H | 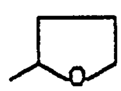 |

| $R^1$ | $R^2$ | $R^3$ | $R^4$ | Het |
|---|---|---|---|---|
| 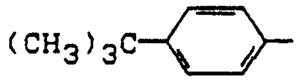 | $CH_3$ | $-CN$ | H | 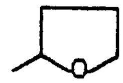 |
| 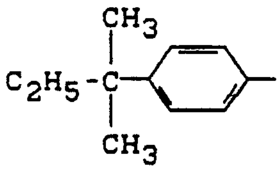 | $CH_3$ | $C_2H_5$ | H | 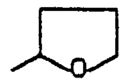 |
| 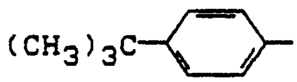 | $CH_3$ | $CH_3$ | H | 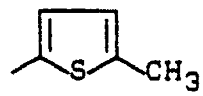 |
| 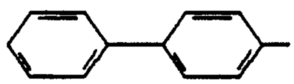 | $CH_3$ | $-(CH_2)_3-CH_3$ | $C_2H_5$ | 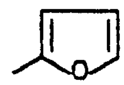 |
| 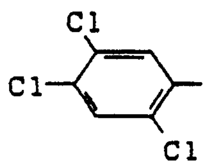 | $CH_3$ | 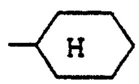 | H | 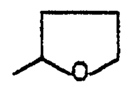 |
| 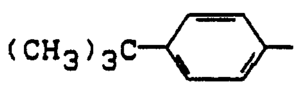 | $CH_3$ | $-CH_2-CH=CH_2$ | H | 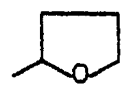 |
| 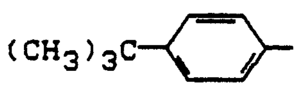 | $CH_3$ | $-CH_2-C\equiv CH$ | H | 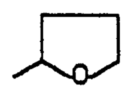 |
| 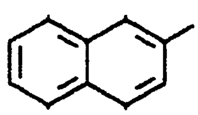 | $CH_3$ | $-(CH_2)_2-CH_3$ | H | 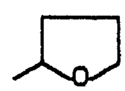 |

| $R^1$ | $R^2$ | $R^3$ | $R^4$ | Het |
|---|---|---|---|---|
| 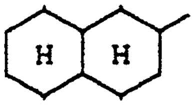 | $CH_3$ | $-(CH_2)_2-CH_3$ | H | 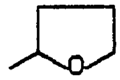 |
| 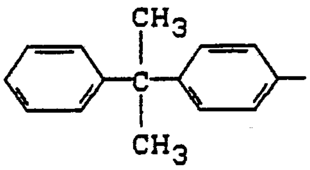 | $CH_3$ | $-(CH_2)_2-CH_3$ | H | 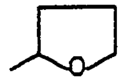 |
| 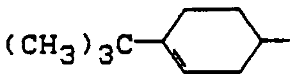 | $CH_3$ | $-(CH_2)_2-CH_3$ | H | 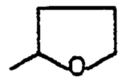 |
| 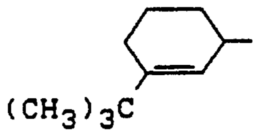 | $CH_3$ | $-(CH_2)_2-CH_3$ | H | 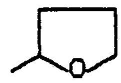 |
| 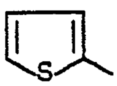 | $CH_3$ | $-(CH_2)_2-CH_3$ | H | 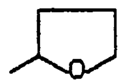 |
| 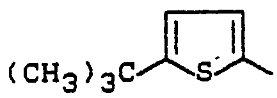 | $CH_3$ | $-(CH_2)_2-CH_3$ | H | 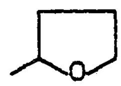 |
| 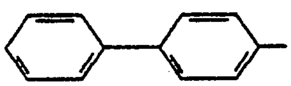 | $CH_3$ | $-(CH_2)_2-CH_3$ | H | 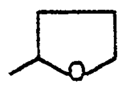 |
| 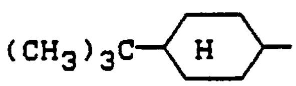 | $CH_3$ | $-CH_2-CH=CH_2$ | H | 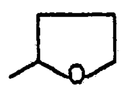 |
| 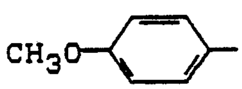 | $CH_3$ | 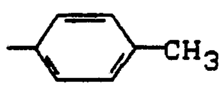 | H | 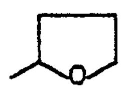 |

| $R^1$ | $R^2$ | $R^3$ | $R^4$ | Het |
|---|---|---|---|---|
| 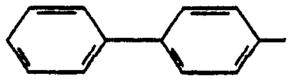 | $CH_3$ | 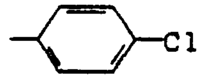 | H | 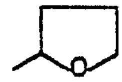 |
| 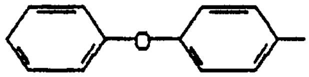 | $CH_3$ | 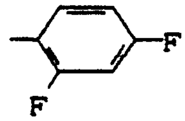 | H | 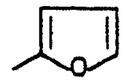 |
| 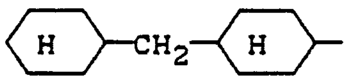 | $CH_3$ | $-(CH_2)_2-CH_3$ | H | 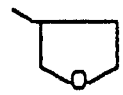 |
| 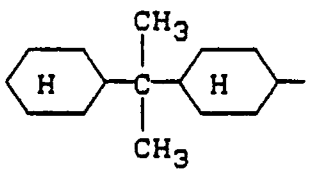 | $CH_3$ | $-(CH_2)_2-CH_3$ | H | 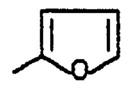 |
| 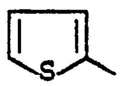 | $CH_3$ | $-(CH_2)_2-CH_3$ | H | 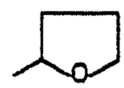 |
| 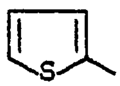 | $CH_3$ | $-(CH_2)_2-CH_3$ | H | 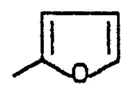 |
| 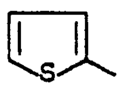 | $CH_3$ | H | H | 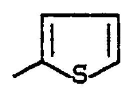 |

Verwendet man beispielsweise 3-(4-t-Butylphenyl)-2-methyl-propionaldehyd und Furanylmethylamin als Ausgangsstoffe, so läßt sich der Reaktionsablauf des erfindungsgemäßen Verfahrens (a) durch das folgende Formelschema darstellen:

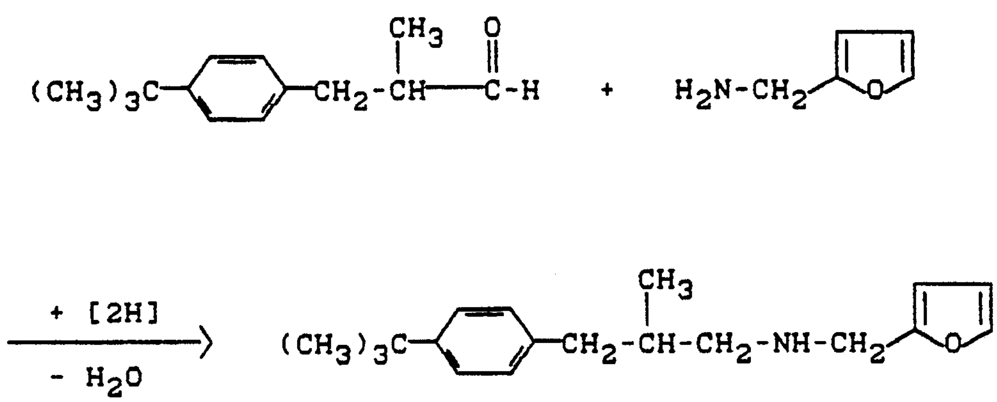


Verwendet man beispielsweise 3-(4-t-Butylphenyl)-2-methyl-N-(2-tetrahydrofuranylmethyl)-propylamin und Allylbromid als Ausgangsstoffe, so läßt sich der Reaktionsablauf des erfindungsgemäßen Verfahrens (b) durch das folgende Formelschema darstellen:

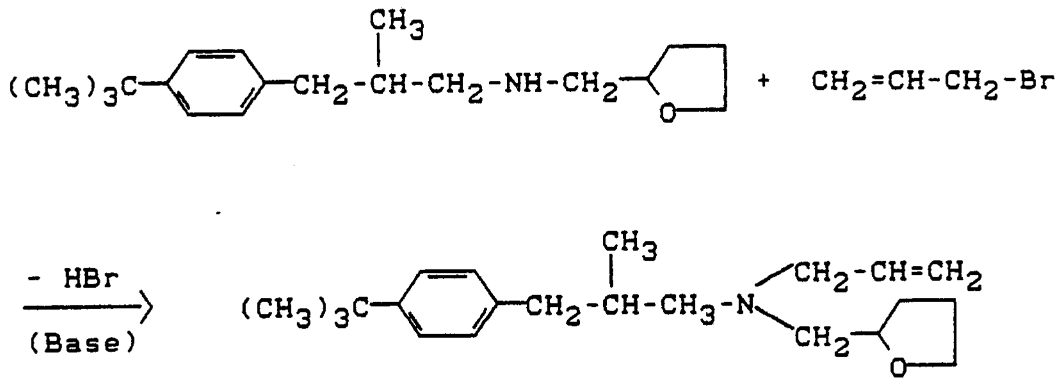


Verwendet man beispielsweise 3-(4-t-Butylphenyl)-2-methyl-N-(2-tetrahydrofuranylmethyl)-propylamin als Ausgangsverbindung, so läßt sich der Reaktionsablauf des erfindungsgemäßen Verfahrens (c) durch das folgende Formelschema darstellen:

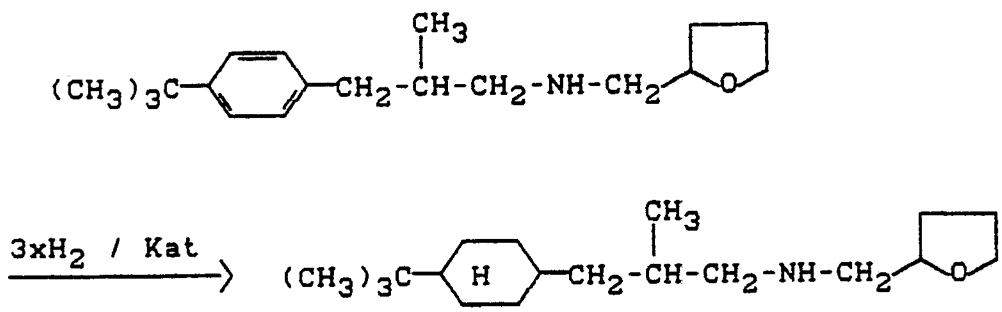


Die zur Durchführung des erfindungsgemäßen Verfahrens (a) als Ausgangsstoffe benötigten substituierten Aldehyde sind durch die Formel (II) allgemeinen definiert. In dieser Formel (II) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Aldehyde der Formel (II) sind größtenteils bekannt (vgl. z. B. Can. PA 1 114 286 vom 15.12.1981; Japan. JP 48/35065 vom 25.10.1973; Jap. Kokai Tokkyo Koho JP 55/36454 vom 14.03.1980; Japan. JP 47/50 095 vom 15.12.1972; Japan. Kokai Tokkyo Koho JP 44/9262 vom 24.01.1979) oder lassen sich in Analogie zu bekannten Verbindungen mit Hilfe allgemein bekannter Verfahren herstellen (vgl. z. B. Tetrahedron 35, 329 -340 [1979]; Chem. Lett. 1977, 423 - 424; J. Org. Chem. 41, 1206 - 1209 [1976]; J. Amer. Chem. Soc. 108, 7361 - 7373 [1986]; Bull. Chem. Soc. Japan 46, 3562 - 3565 [1973] sowie Org. Prep. Proced. Int. 14, 9 - 20 [1982]).

Die zur Durchführung des erfindungsgemäßen Verfahrens (a) weiterhin als Ausgangsstoffe benötigten Amine sind durch die Formel (III) allgemein definiert. In dieser Formel (III) stehen $R^3$, $R^4$ und Het vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (III) sind allgemein bekannte Verbindungen der organischen Chemie oder erhältlich in Analogie zu allgemein bekannten Verfahren (vgl. z. B. J. Prakt. Chem. 317, 897 - 906 [1975]; DE-OS 27 57 922; Ind. J. Chem. B; 193; 310 - 312 [1980]; Belg. BE 883 713 vom 09.12.1980 oder Japan. Kokai Tokkyo Koho JP 57/2274 vom 07.01.1982).

Die zur Durchführung des erfindungsgemäßen Verfahrens (b) als Ausgangsstoffe benötigten substituierten Propylamine sind durch die Formel (Ia) allgemein definiert. In dieser Formel (Ia) stehen $R^1$, $R^2$, $R^4$ und Het vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Propylamine der Formel (Ia) sind erfindungsgemäße Verbindungen und erhältlich mit Hilfe des erfindungsgemäßen Verfahrens (a) oder (c).

Die zur Durchführung des erfindungsgemäßen Verfahrens (b) weiterhin als Ausgangsstoffe benötigten Alkylie rungsmittel sind durch die Formel (IV) allgemein definiert. In dieser Formel (IV) steht $R^{3-1}$ vorzugsweise für geradkettiges oder verzweigtes Alkyl mit 1 bis 8 Kohlenstoffatomen, für jeweils geradkettiges oder verzweigtes Alkenyl oder Alkinyl mit jeweils 3 bis 8 Kohlenstoffatomen, für jeweils geradkettiges oder verzweigtes Alkoxyalkyl, Hydroxyalkoxyalkyl oder Dialkoxyalkyl mit jeweils 1 bis 4 Kohlenstoffatomen in den einzelnen Alkylteilen, für geradkettiges oder verzweigtes Hydroxyalkyl mit 2 bis 8 Kohlenstoffatomen, für jeweils gegebenenfalls einfach bis mehrfach, gleich oder verschieden durch geradkettiges oder verzweigtes Alkyl mit 1 bis 4 Kohlenstoffatomen substituiertes Cycloalkyl mit 3 bis 7 Kohlenstoffatomen, Cycloalkenyl mit 3 bis 7 Kohlenstoffatomen, Cycloalkylalkyl mit 3 bis 7 Kohlenstoffatomen im Cycloalkylteil und 1 bis 8 Kohlenstoffatomen im geradkettigen oder verzweigten Alkylteil, Cycloalkenylalkyl mit 3 bis 7 Kohlenstoffatomen im Cycloalkenylteil und 1 bis 8 Kohlenstoffatomen im geradkettigen oder verzweigten Alkylteil, Furanylmethyl oder Tetrahydrofuranylmethyl oder für gegebenenfalls einfach bis mehrfach, gleich oder verschieden substituiertes Phenylalkyl mit 1 bis 4 Kohlenstoffatomen im geradkettigen oder verzweigten Alkylteil, wobei als Phenylsubstituenten infrage kommen: Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oer Halogenalkylthio mit jeweils 1 bis 4 Kohlenstoffatomen und 1 bis 9 gleichen oder verschiedene Halogenatomen;

$R^{3-1}$ steht besonders bevorzugt für Methyl, Ethyl, n-oder i-Propyl, n-, i-, s-oder t-Butyl, für jeweils geradkettiges oder verzweigtes Pentyl, Hexyl, Heptyl oder Octyl, für Allyl, Butenyl, Propargyl, Butinyl, für Methoxyethyl, Methoxypropyl, Ethoxyethyl, Ethoxypropyl, Propoxypropyl, Dimethoxyethyl, Hydroxyethyl, Hydroxypropyl, Hydroxyethoxyethyl, für jeweils gegebenenfalls ein-bis dreifach, gleich oder verschieden substituiertes Cyclohexyl, Cyclohexenyl, Cyclopentyl, Cyclohexylmethyl, Cyclohexylethyl, Cyclohexenylmethyl, Cyclopentylmethyl, Furanylmethyl oder Tetrahydrofuranylmethyl, wobei als Substituenten jeweils infrage kommen: Methyl, Ethyl n-oder i-Propyl sowie n-, i-, s-oder t-Butyl und außerdem für jeweils gegebenenfalls ein-bis dreifach, gleich oder verschieden substituiertes Benzyl oder Phenylethyl, wobei als Phenylsubstituenten jeweils infrage kommen: Fluor, Chlor, Brom, Methyl, Ethyl, n-oder i-Propyl, Methoxy, Ethoxy, Methylthio, Trifluormethyl, Trifluormethoxy oder Trifluormethylthio;

$R^{3-1}$ steht ganz besonders bevorzugt für Methyl, Ethyl, n-oder i-Propyl, n-, i-oder s-Butyl, n-Pentyl, n-Hexyl, n-Octyl, Allyl, Propargyl, Methoxyethyl, Methoxypropyl, Ethoxyethyl oder Ethoxypropyl.

E steht vorzugsweise für Halogen, insbesondere für Chlor, Brom oder lod, oder für jeweils gegebenenfalls substituiertes Alkylsulfonyloxy, Alkoxysulfonyloxy oder Arylsulfonyloxy, wie beispiels weise Methansulfonyloxy, Methoxysulfonyloxy oder p-Toluolsulfonyloxy.

Die Alkylierungsmittel der Formel (IV) sind allgemein bekannte Verbindungen der organischen Chemie.

Die zur Durchführung des erfindungsgemäßen Verfahrens (c) als Ausgangsstoffe benötigten substituierten Propylamine sind durch die Formel (Ib) allgemein definiert. In dieser Formel (Ib) stehen $R^2$, $R^3$, $R^4$ und Het vorzugsweise für diejenigen Reste, die bereits im Zusammenhang mit der Beschreibung der erfindungsgemäßen Stoffe der Formel (I) als bevorzugt für diese Substituenten genannt wurden.

$R^{1-1}$ steht vorzugsweise für jeweils gegebenenfalls einfach bis mehrfach, gleich oder verschieden substituiertes Phenyl oder Naphthyl, wobei als Substituenten jeweils infrage kommen: Halogen, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils gegebenenfalls durch geradkettiges oder verzweigtes Alkyl mit 1 bis 6 Kohlenstoffatomen einfach bis mehrfach, gleich oder verschieden substituiertes Cycloalkyl mit 3 bis 7 Kohlenstoffatomen, Aryl oder Aryloxy mit 6 bis 10 Kohlenstoffatomen sowie Arylalkyl mit 6 bis 10 Kohlenstoffatomen im Arylteil und 1 bis 6 Kohlenstoffatomen im geradkettigen oder verzweigten Alkylteil;

$R^{1-1}$ steht besonders bevorzugt für jeweils gegebenenfalls ein-bis dreifach, gleich oder verschieden substituiertes Phenyl oder Naphthyl, wobei als Substituenten jeweils infrage kommen: Fluor, Chlor, Brom, Methyl, Ethyl, n-oder i-Propyl, n-, i-, s-oder t-Butyl, jeweils geradkettiges oder verzweigtes Pentyl, Hexyl, Heptyl, Octyl, Methoxy, Ethoxy, n-oder i-Propoxy, n-, i-, s-oder t-Butoxy, Methylthio, Ethylthio, n-oder i-Propylthio, Trifluormethyl, Trifluormethoxy, Trifluormethylthio, Cyclohexyl, Phenyl, Phenoxy, Benzyl, 1-Phenylethyl, 1-Phenylpropyl, 2-Phenyl-2-propyl sowie 2-Phenyl-2-butyl.

Die substituierten Propylamine der Formel (Ia) sind erfindungsgemäße Verbindungen und erhältlich mit Hilfe des erfindungsgemäßen Verfahrens (a).

Als Verdünnungsmittel zur Durchführung des erfindungsgemäßen Verfahrens (a) kommen inerte organische Lösungsmittel infrage. Vorzugsweise verwendet man polare oder dipolar aprotische Lösungsmittel, so z.B. Alkohole, wie Methanol, Ethanol, n-oder i-Propanol, Ester, wie Essigsäureethylester oder Ether, wie Diethylether, Dioxan oder Tetrahydrofuran.

Als Reduktionsmittel zur Durchführung des erfindungsgemäßen Verfahrens (a) kommen alle üblicherweise für der artige reduktive Aminierungen verwendbaren Reduktionsmittel infrage. Vorzugsweise verwendet man molekularen Wasserstoff in Gegenwart eines geeigneten Hydrierkatalysators oder Ameisensäure.

Als Katalysatoren zur Durchführung des erfindungsgemäßen Verfahrens (a) kommen übliche Hydrierkatalysatoren in Frage. Vorzugsweise verwendet man Edelmetallkatalysatoren wie Palladium, Platin oder Platinoxid gegebenenfalls in Gegenwart eines geeigneten Trägers, wie beispielsweise Kohlenstoff, Aluminiumoxid oder Siliciumdioxid.

Die Reaktionstemperaturen können bei der Durchführung des erfindungsgemäßen Verfahrens (a) in einem größeren Bereich variiert werden. Im allgemeinen arbeitet man bei Temperaturen zwischen 20 °C und 250 °C, vorzugsweise bei Temperaturen zwischen 50 °C und 150 °C.

Das erfindungsgemäße Verfahren (a) wird gegebenenfalls unter erhöhtem Druck durchgeführt. Im allgemeinen arbeitet man in einem Druckbereich zwischen 1 und 150 bar, vorzugsweise zwischen 20 und 100 bar.

Zur Durchführung des erfindungsgemäßen Verfahrens (a) setzt man pro Mol an substituierten Aldehyd der Formel (II) im allgemeinen 1,0 bis 3,0 Mol, vorzugweise äquimolare Mengen an Amin der Formel (III), 1,0 bis 20,0 Mol an Reduktionsmittel und gegebenenfalls 0,001 bis 0,1 Mol an Katalysator ein.

Die Reaktionsdurchführung, Aufarbeitung und Isolierung der Reaktionsprodukte erfolgt nach allgemein bekannten, üblichen Methoden (vgl. z.B. "Organikum" 15. Auflage, S. 542, VEB Deutscher Verlag der Wissenschaften Berlin 1981).

In einer Variante zur Reaktionsdurchführung des erfindungsgemäßen Verfahrens (a) ist es auch möglich, die als Ausgangsverbindungen infrage kommenden substituierten Aldehyde der Formel (II) in einer vorgelagerten Reaktion direkt im Reaktionsgefäß herzustellen ("Eintopfreaktion"), indem man allgemein bekannte Aldehyde der Formel (V),

$$R^1-\overset{O}{\overset{\|}{C}}-H \qquad (V)$$

in welcher

$R^1$ die oben angegebene Bedeutung hat,

mit ebenfalls allgemein bekannten Aldehyden der Formel (VI),

$$R^2-CH_2-\overset{O}{\overset{\|}{C}}-H \qquad (VI)$$

in welcher

$R^2$ die oben angegebene Bedeutung hat,

in Gegenwart einer Base wie beispielsweise Natriumhydroxid und gegebenenfalls in Gegenwart eines Verdünnungsmittels wie beispielsweise Methanol bei Temperaturen zwischen 0 °C und 80 °C kondensiert ("Aldol-Kondensation") und das so erhältliche Reaktionsgemisch mit Aminen der Formel (III) in Gegenwart eines Reduktionsmittels gemäß dem erfindungsgemäßen Verfahren (a) direkt weiter umsetzt (vgl. hierzu beispielsweise DE-OS 31 05 446).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzo, Toluol, Xylol, Chlorbenzol, Petrolether, Hexan, Cyclohexan, Dichlomethan, Chloroform, Tetrachlorkohlenstoff, Ether, wie Diethylether, Dioxan, Tetrahydrofuran oder Ethylenglykoldimethyl oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (b) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-dimethylammoniummethylsulfat, Dimethyl-$C_{12}/C_{14}$-alkyl-benzylammoniumchlo rid, Tetrabutylammoniumhydroxid, 15-Krone-5, 18-Krone-6, Triethylbenzylammoniumchlorid, Trimethylbenzylammoniumchlorid.

Als Reaktionshilfsmittel zur Durchführung des erfindungsgemäßen Verfahrens (b) kömmen alle üblicherweise verwendbaren anorganischen und organischen Basen infrage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen - 20 °C

und + 150 °C. vorzugsweise bei Temperaturen zwischen 0 °C und + 100 °C.

Zur Durchführung des erfindungsgemäßen Verfahrens (b) setzt man pro Mol an substituiertem Propylamin der Formel (Ia) im allgemeinen 1,0 bis 20,0 Mol, vorzugsweise 1,0 bis 15,0 Mol an Alkylierungsmittel der Formel (IV) und gegebenenfalls 1,0 bis 3,0 Mol, vorzugsweise 1,0 bis 2,0 Mol an Reaktionshilfmittel sowie gegebenenfalls 0,01 bis 1,0 Mol an Phasentransferkatalysator ein. Die Reaktionsdurchführung, Aufarbeitung und Isolierung der Reaktionsprodukte der Formel (I) erfolgt in allgemein üblicher Art und Weise.

Als Verdünnungsmittel zur Durchführung des erfindungsgemäßen Verfahrens (c) kommen inerte organische Lösungsmittel infrage. Hierzu gehören vorzugsweise aliphatische Kohlenwasserstoffe, wie Petrolether, Hexan oder Cyclohexan; Ether, wie Diethylether, Dioxan, Tetrahydrofuran oder Ethylenglykoldimethyl oder -diethylether; Ester, wie Essigsäureethylester; Sulfoxide, wie Dimethylsulfoxid; sowie Alkohole, wie Methanol oder Ethanol.

Als Katalysatoren zur Durchführung des erfindungsgemäßen Verfahrens (c) kommen übliche Hydrierkatalysatoren in Frage, Vorzugsweise verwendet man Edelmetall-, Edelmetalloxid-oder Edelmetallhydroxid-Katalysatoren bzw. sogenannte Raney-Katalysatoren, wie insbesondere Platin, Platinoxid, Nickel und Ruthenium, gegebenenfalls auf einen geeigneten Träger wie Kohlenstoff, Aluminiumoxid oder Siliciumdioxid.

Die Reaktionstemperaturen können bei der Durchführung des erfindungsgemäßen Verfahrens (c) in einem größeren Bereich variiert werden. Im allgemeinen arbeitet man bei Temperaturen zwischen 20 °C und 250 °C, vorzugsweise bei Temperaturen zwischen 20 °C und 200 °C.

Das erfindungsgemäße Verfahren (c) kann bei Normaldruck oder auch bei erhöhtem Druck durchgeführt werden. Im allgemeinen arbeitet man zwischen 1 atm und 300 atm, vorzugsweise zwischen 1 atm und 200 atm.

Zur Durchführung des erfindungsgemäßen Verfahrens (c) setzt man pro Mol an substituiertem Proypylamin der Formel (Ib) 0,001 bis 0,1 Mol an Hydrierkatalysator zu und gibt in einem Autoklaven Wasserstoff zu, bis sich der erforderliche Druck eingestellt hat. Die Reaktionsdurchführung, Aufarbeitung und Isolierung der Reaktionsprodukte der Formel (I) erfolgt in Analogie zu allgemein bekannten Verfahren (vgl. z.B. DE-OS 27 52 135).

Zur Herstellung von pflanzenverträglichen Säureadditionssalzen der Verbindungen der Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi-und trifunktionelle Carbonsäuren und Hydroxycarbonsäuren, wie z.B. Ameisensäure, Essigsäure, Maleinsäure, Bernsteinsäure, Fumarsäure, Weinsäure, Zitronensäure, Salicylsäure, Sorbinsäure, Milchsäure; Sulfonsäuren, wie z.B. p-Toluolsulfonsäure und 1,5-Naphthalindisulfonsäure sowie Saccharin.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;

Phytophthora-Arten, wie beispielsweise Phytophthora infestans;

Pseudoperonospora-Arten, wie beispielsweise Pseudoperonospora humuli oder Pseudoperonospora cubensis;

Plasmopara-Arten, wie beispielsweise Plasmopara viticola;

Peronospora-Arten, wie beispielsweise Peronospora pisi oder P. brassicae;

Erysiphe-Arten, wie bêispielsweise Erysiphe graminins;

Sphaerotheca-Arten, wie beispielsweise Sphaerotheca fuliginea;

Podosphaera-Arten, wie beispielsweise Podosphaera leucotricha;

Venturia-Arten, wie beispielsweise Venturia inaequalis;

Pyrenophora-Arten, wie beispielsweise Pyrenophora teres oder P. graminea (Konidienform: Drechslera, Syn: Helminthosporium);

Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilage nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger der Braunspelzigkeit des Weizens (Leptrosphaeria nodorum), oder gegen Getreiderost-und Getreidemehltauerreger sowie zur Bekämpfung von Reiskrankheiten wie beispielsweise gegen den Erreger der Reisfleckenkrankheit (Pyricularia oryzae) einsetzen.

Daneben zeigen die erfindungsgemäßen Wirkstoffe auch eine insektizide Wirksamkeit und lassen sich beispielsweise zur Bekämpfung von Blattinsektiziden einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solch Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder -schaumerzeugende Mittel kommen in Frage: z.B. nichtionogene und anionische Emulgatoren, wie Polyoxyethylen-Fettsäure-Ester, Polyoxy ethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formu lierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1:

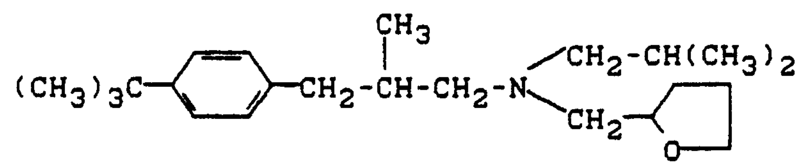

(Verfahren a)

Eine Lösung aus 4,7 g (0,03 Mol) N-Isobutyl-tetrahydrofuran-2-yl-methylamin (vgl. DE-P 1 278 443) und 6,1 g (0,03 Mol) 3-(4-t-Butylphenyl)-2-methyl-propionaldehyd (vgl. z.B. EP 58 326) in 100 ml Methanol wird zusammen mit 2 g Palladium auf Aluminiumoxid (1 %) bei 100 °C und 50 bar Wasserstoffdruck 3 Stunden hydriert. Zur Aufarbeitung wird der Katalysator abfiltriert und das Lösungsmittel abdestilliert.
Man erhält 9,5 g (92 % der Theorie) an [3-(4-t-Butylphenyl)-2-methyl]-N-isobutyl-N-tetrahydrofuran-2-yl-methyl-propylamin als Öl von einer gaschromatographisch bestimmten Reinheit von 95 %.
$^1$H-NMR ($CDCl_3$/Tetramethylsilan) $\delta$ = 3,65-4,0 (m, 3H); 1,3 (s, 9H) ppm.

In entsprechender Weise und gemäß den allgemeinen Angaben zur Herstellung erhält man die folgenden substituierten Propylamine der allgemeinen Formel (I):

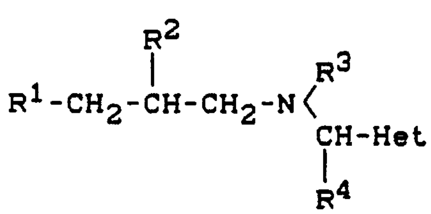 (I)

| Bsp. Nr. | $R^1$ | $R^2$ | $R^3$ | $R^4$ | Het | physikalische Eigenschaften |
|---|---|---|---|---|---|---|
| 2 | $(CH_3)_3C$- 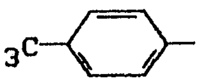 | $CH_3$ | $CH_3$ | H | 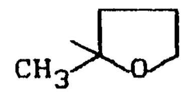 | $^1H$-NMR*): 1,3; 3,75-3,9 |
| 3 | $(CH_3)_3C$- 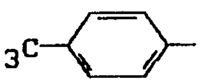 | $CH_3$ | $CH_3-(CH_2)_2-$ | H | 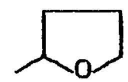 | $^1H$-NMR*): 1,3; 3,7-4,0 |
| 4 | $(CH_3)_3C$- 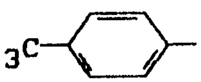 | $CH_3$ | $CH_3$ | H | 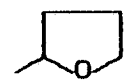 | $^1H$-NMR*): 1,3; 3,65-4,05 |
| 5 | $(CH_3)_3C$- 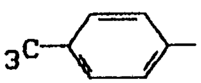 | $CH_3$ | $(CH_3)_2CH-$ | H | 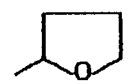 | $^1H$-NMR*): 1,3; 3,70-3,95 |
| 6 | $(CH_3)_3C$- 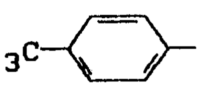 | $CH_3$ | 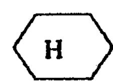 $-CH_2-$ | H | 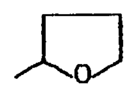 | $^1H$-NMR*); 1,3; 3,7-4,0 |

| Bsp. Nr. | $R^1$ | $R^2$ | $R^3$ | $R^4$ | Het | physikalische Eigenschaften |
|---|---|---|---|---|---|---|
| 7 | 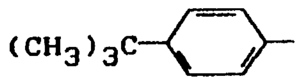 | $CH_3$ | $CH_3$ | H | 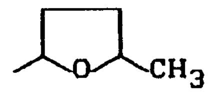 | $^1$H-NMR*): 1,3; 3,9-4,0 |
| 8 | 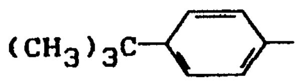 | $CH_3$ | 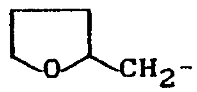 | H | 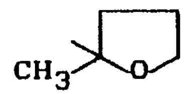 | $^1$H-NMR*); 1,3; 3,6-4,0 |
| 9 | 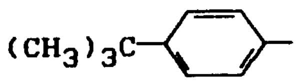 | $CH_3$ | 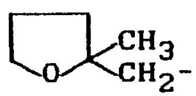 | H | 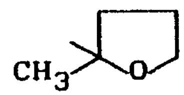 | $^1$H-NMR*): 1,3; 3,7-3,9 |
| 10 | 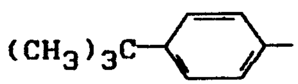 | $CH_3$ | 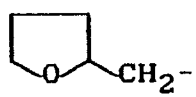 | H | 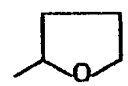 | $^1$H-NMR*): 1,3; 3,65-4,0 |
| 11 | 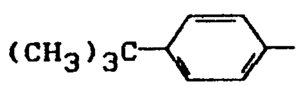 | $CH_3$ | $CH_3(CH_2)_2-$ | H | 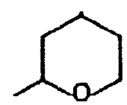 | $^1$H-NMR*): 1,3; 3,25-3,45 |

| Bsp. Nr. | $R^1$ | $R^2$ | $R^3$ | $R^4$ | Het | physikalische Eigenschaften |
|---|---|---|---|---|---|---|
| 12 | $(CH_3)_3C$ 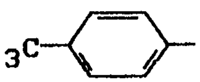 | $CH_3$ | H | H | 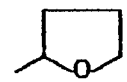 | $^1H$-NMR*): 1,3; 3,7-4,05 |
| 13 | $(CH_3)_3C$ 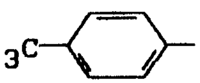 | $CH_3$ | H | H | 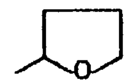 | Fp. 142 °C (Hydrochlorid) |
| 14 | $(CH_3)_3C$ 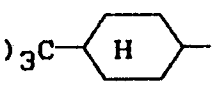 | $CH_3$ | $-(CH_2)_2-CH_3$ | H | 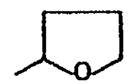 | $^1H$-NMR*): 0,8; 3,7-4,1 |
| 15 | $(CH_3)_3C$ 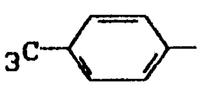 | $CH_3$ | $-C_2H_5$ | H | 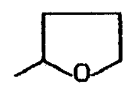 | $^1H$-NMR*): 1,4; 3,7-4,05 |
| 16 | $(CH_3)_3C$ 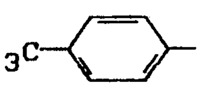 | $CH_3$ | $-(CH_2)_3-CH_3$ | H | 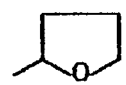 | $^1H$-NMR*): 1,3;3,65-4,0 |

| Bsp. Nr. | $R^1$ | $R^2$ | $R^3$ | $R^4$ | Het | physikalische Eigenschaften |
|---|---|---|---|---|---|---|
| 17 | $(CH_3)_3C$ 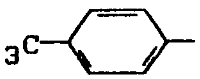 | $CH_3$ | $-(CH_2)_7-CH_3$ | H | 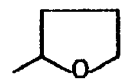 | $^1H$-NMR*): 1,3; 3,6-4,0 |
| 18 | $(CH_3)_3C$ 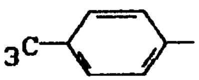 | $CH_3$ | 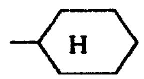 | H | 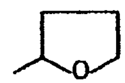 | $^1H$-NMR*): 1,3; 3,65-3,95 |
| 19 | $(CH_3)_3C$ 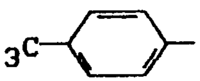 | $CH_3$ | $CH_3$<br>$-CH-C_2H_5$ | H | 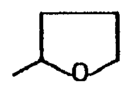 | $^1H$-NMR*): 1,3; 3,7-3,95 |
| 20 | $(CH_3)_3C$ 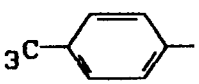 | $CH_3$ | $-(CH_2)_2-CH_3$ | H | 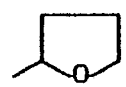 x 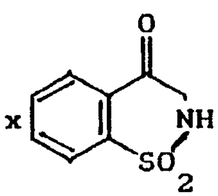 | $^1H$-NMR*): 1,3; 7,1; 7,3; 7,6; 7,8; |

| Bsp. Nr. | $R^1$ | $R^2$ | $R^3$ | $R^4$ | Het | physikalische Eigenschaften |
|---|---|---|---|---|---|---|
| 21 | 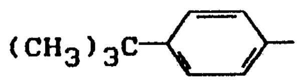 | $CH_3$ | 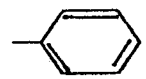 | H | 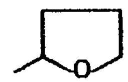 | $^1$H-NMR*): 1,3; 3,7-4,15 |
| 22 | 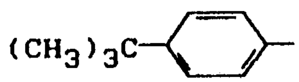 | $CH_3$ | $-CH_2-CH_2-OCH_3$ | H | 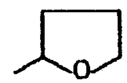 | $^1$H-NMR*): 1,3; 3,65-4,0 |
| 23 | 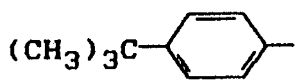 | $CH_3$ | $-(CH_2)_3-OCH_3$ | H | 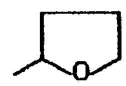 | $^1$H-NMR*): 1,3; 3,65-4,0 |
| 24 | 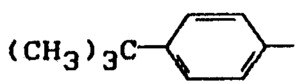 | $CH_3$ | $-(CH_2)_2-CH_3$ | H | 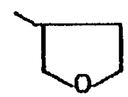 | $^1$H-NMR*): 1,3; 3,5-3,9 |
| 25 | 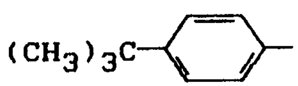 | $CH_3$ | $-(CH_2)_3-O_2H_5$ | H | 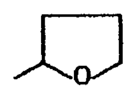 | $^1$H-NMR*): 1,3; 3,7-4,0 |

| Bsp. Nr. | $R^1$ | $R^2$ | $R^3$ | $R^4$ | Het | physikalische Eigenschaften |
|---|---|---|---|---|---|---|
| 26 | 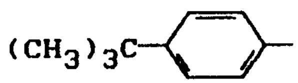 | $CH_3$ | $-CH_2-CH=CH_2$ | H | 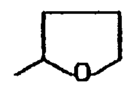 | $^1$H-NMR*): 1,3; 3,7-4,0 |
| 27 | 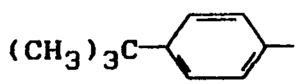 | $CH_3$ | 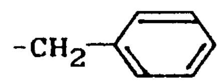 | H | 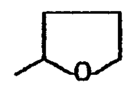 | $^1$H-NMR*): 1,3; 3,65-4,05 |
| 28 | 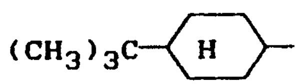 | $CH_3$ | $-C_2H_5$ | H | 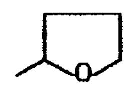 | $^1$H-NMR*): 0,8; 3,6-4,05 |
| 29 | 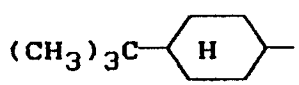 | $CH_3$ | $-(CH_2)_3-CH_3$ | H | 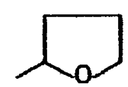 | $^1$H-NMR*): 0,8; 3,65-4,0 |
| 30 | 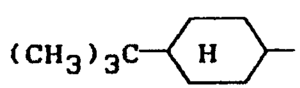 | $CH_3$ | 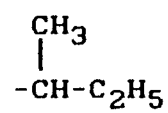 | H | 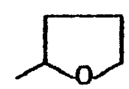 | $^1$H-NMR*): 0,8; 3,65-3,9 |

| Bsp. Nr. | $R^1$ | $R^2$ | $R^3$ | $R^4$ | Het | physikalische Eigenschaften |
|---|---|---|---|---|---|---|
| 31 | 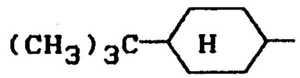 | $CH_3$ | $-(CH_2)_3-OCH_3$ | H | 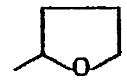 | $^1H$-NMR*): 0,8; 3,65-3,95 |
| 32 | 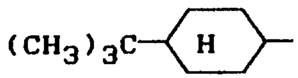 | $CH_3$ | $-(CH_2)_2-CH_3$ | H | 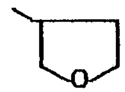 | $^1H$-NMR*): 0,8; 3,7-3,9 |
| 33 | 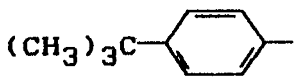 | $CH_3$ | H | H | 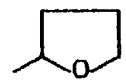 x 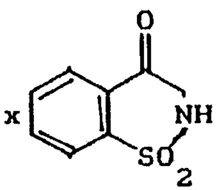 | $^1H$-NMR*): 1,3; 7,1; 7,3; 7,6; 7,8; |
| 34 | 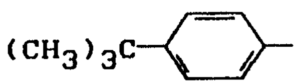 | $CH_3$ | $C_2H_5$ | H | 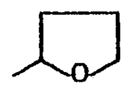 x 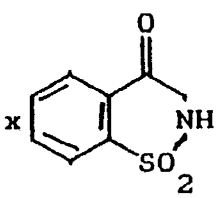 | $^1H$-NMR*): 1,3; 7,1; 7,3; 7,6; 7,8; |

| Bsp. Nr. | $R^1$ | $R^2$ | $R^3$ | $R^4$ | Het | physikalische Eigenschaften |
|---|---|---|---|---|---|---|
| 35 | 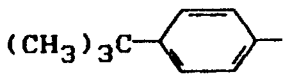 | $CH_3$ | $-(CH_2)_3-CH_3$ | H | 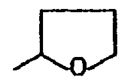 x 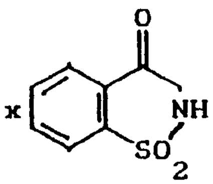 | $^1H$-NMR*): 1,3; 7,1; 7,3; 7,6; 7,8; |
| 36 | 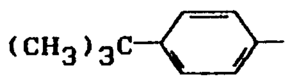 | $CH_3$ | $-(CH_2)_7-CH_3$ | H | 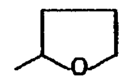 x 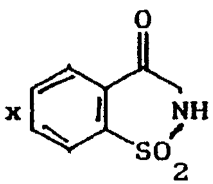 | $^1H$-NMR*): 1,3; 7,1; 7,3; 7,6; 7,8; |
| 37 | 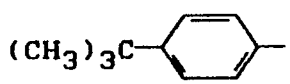 | $CH_3$ | 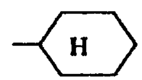 | H | 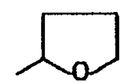 x 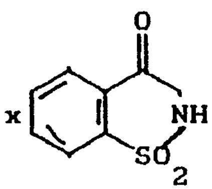 | $^1H$-NMR*): 1,3; 7,1; 7,3; 7,6; 7,8; |
| 38 | 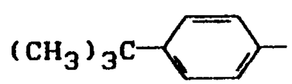 | $CH_3$ | $CH_3$<br>\|<br>$-CH-C_2H_5$ | H | 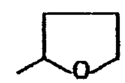 x 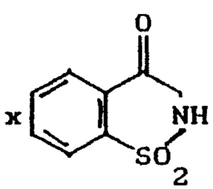 | $^1H$-NMR*): 1,3; 7,1; 7,3; 7,6; 7,8; |

| Bsp. Nr. | $R^1$ | $R^2$ | $R^3$ | $R^4$ | Het | physikalische Eigenschaften |
|---|---|---|---|---|---|---|
| 39 | 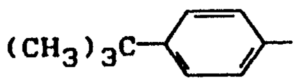 | $CH_3$ | $-(CH_2)_3-OCH_3$ | H | 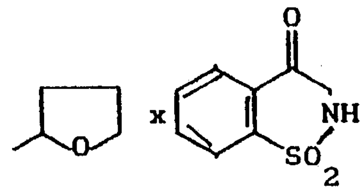 | $^1$H-NMR*): 1,3; 7,1; 7,3; 7,6; 7,8; |
| 40 | 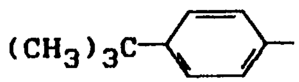 | $CH_3$ | $-(CH_2)_2-CH_3$ | H | 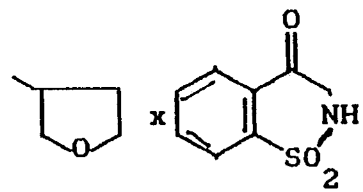 | $^1$H-NMR*): 1,3; 7,1; 7,3; 7,6; 7,8; |
| 41 | 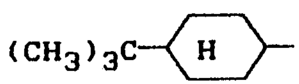 | $CH_3$ | $-(CH_2)_2-OCH_3$ | H | 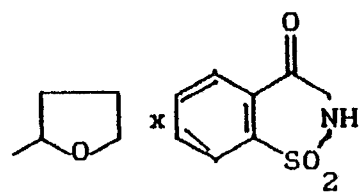 | $^1$H-NMR*): 0,8; 3,6 - 4,0 |
| 42 | 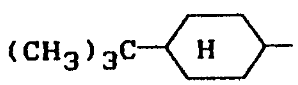 | $CH_3$ | $-(CH_2)_3-OC_2H_5$ | H | 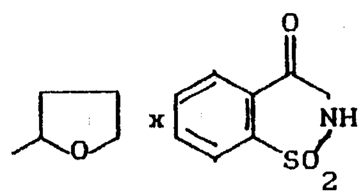 | $^1$H-NMR*): 0,8; 3,7 - 4,0 |

| Bsp. Nr. | $R^1$ | $R^2$ | $R^3$ | $R^4$ | Het | physikalische Eigenschaften |
|---|---|---|---|---|---|---|
| 43 | $(CH_3)_3C$- 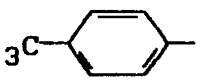 | $CH_3$ | $-CH_2-CH=CH_2$ | H | 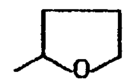 x 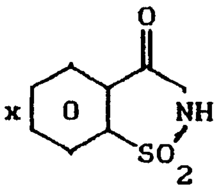 | $^1$H-NMR*): 1,3; 7,1; 7,3; 7,6; 7,8; |
| 44 | $(CH_3)_3C$- 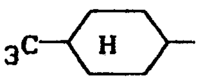 | $CH_3$ | $-(CH_2)_2-CH_3$ | H | 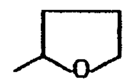 x 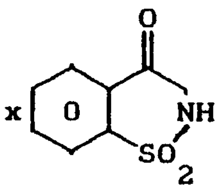 | $^1$H-NMR*): 0,8; 7,6; 7,8 |
| 45 | $(CH_3)_3C$- 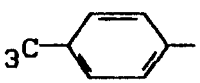 | $CH_3$ | 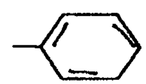 | H | 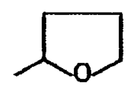 x 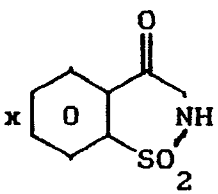 | $^1$H-NMR*): 1,3; 6,6; 7,0 - 7,3; 7,6 - 7,9 |
| 46 | $(CH_3)_3C$- 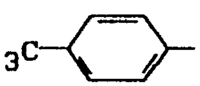 | $CH_3$ | $-(CH_2)_2-OCH_3$ | H | 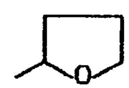 x 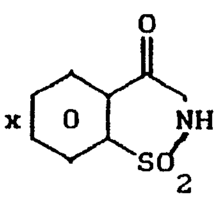 | $^1$H-NMR*): 1,3; 7,1; 7,3; 7,6; 7,8; |

| Bsp. Nr. | $R^1$ | $R^2$ | $R^3$ | $R^4$ | Het | physikalische Eigenschaften |
|---|---|---|---|---|---|---|
| 47 | $(CH_3)_3C$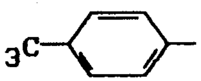 | $CH_3$ | $-(CH_2)_3-OC_2H_5$ | H | 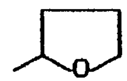 x 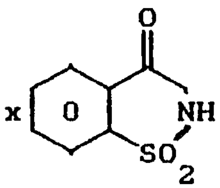 | $^1H$-NMR*): 1,3; 7,1; 7,3; 7,6; 7,8 |
| 48 | $(CH_3)_3C$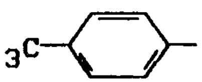 | $CH_3$ | $-CH_2-C{\equiv}CH$ | H | 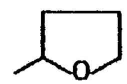 | $^1H$-NMR*): 1,3; 3,7 - 4,0 |
| 49 | $(CH_3)_3C$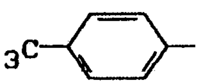 | $CH_3$ | $-CH_2$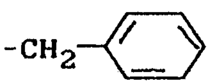 | H | 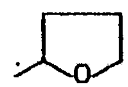 x 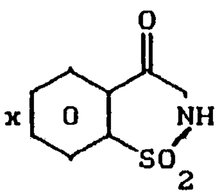 | $^1H$-NMR*): 1,3; 7,1; 7,3-7,4; 7,6; 7 |
| 50 | $(CH_3)_3C$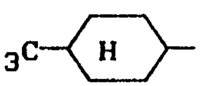 | $CH_3$ | $-(CH_2)_2-OCH_3$ | H | 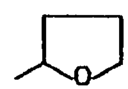 x 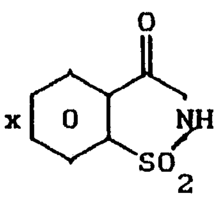 | $^1H$-NMR*): 0,8; 7,6; 7,8 |

| Bsp. Nr. | $R^1$ | $R^2$ | $R^3$ | $R^4$ | Het | physikalische Eigenschaften |
|---|---|---|---|---|---|---|
| 51 | 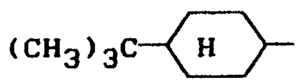 | $CH_3$ | $-(CH_2)_3-OC_2H_5$ | H | 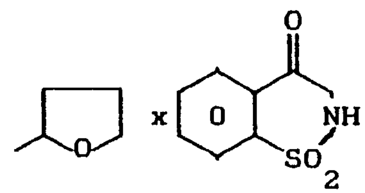 | $^1H$-NMR*): 0,8; 7,6; 7,8 |
| 52 | 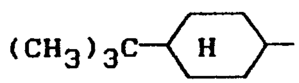 | $CH_3$ | 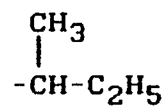 | H | 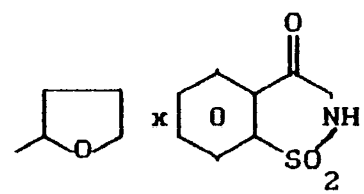 | $^1H$-NMR*): 0,8; 7,6; 7,8 |
| 53 | 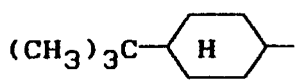 | $CH_3$ | $-(CH_2)_2-OCH_3$ | H | 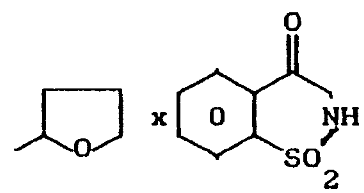 | $^1H$-NMR*): 0,8; 7,6; 7,8 |
| 54 | 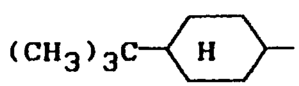 | $CH_3$ | $-(CH_2)_2-CH_3$ | H | 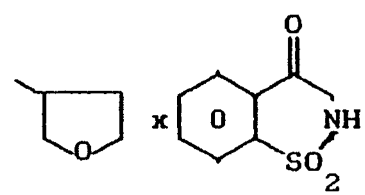 | $^1H$-NMR*): 0,8; 7,6; 7,8 |

| Bsp. Nr. | $R^1$ | $R^2$ | $R^3$ | $R^4$ | Het | physikalische Eigenschaften |
|---|---|---|---|---|---|---|
| 55 | $(CH_3)_3C$ 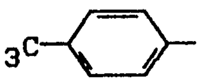 | $CH_3$ | $-(CH_2)_2-CH_3$ | H | 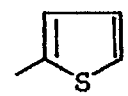 | $^1$H-NMR*): 6,7; 6,8; 7,05;7,15 7,3 |
| 56 | $(CH_3)_3C$ 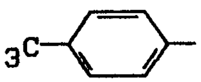 | $CH_3$ | $-CH_2-C{\equiv}CH$ | H | 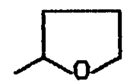 x 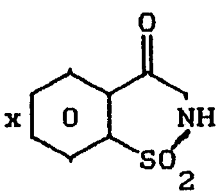 | $^1$H-NMR*): 1,3; 7,1; 7,3; 7,6; 7,8 |
| 57 | $(CH_3)_3C$ 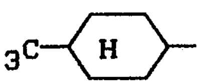 | $CH_3$ | $CH_3$ | H | 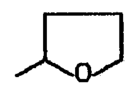 | $^1$H-NMR*): 1,3; 3,7 - 4,0 |
| 58 | $(CH_3)_3C$ 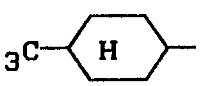 | $CH_3$ | $CH_3$ | H | 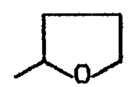 x 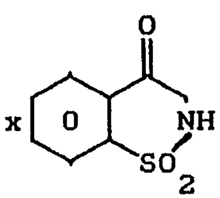 | $^1$H-NMR*): 0,8; 7,6; 7,8 |

| Bsp. Nr. | $R^1$ | $R^2$ | $R^3$ | $R^4$ | Het | physikalische Eigenschaften |
|---|---|---|---|---|---|---|
| 59 | $(CH_3)_3C$ 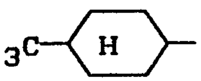 | $CH_3$ | $C_2H_5$ | H | 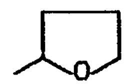 x 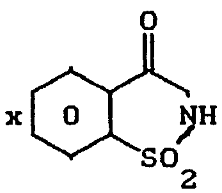 | $^1$H-NMR*): 0,8; 7,6; 7,8 |
| 60 | $(CH_3)_3C$ 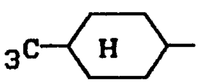 | $CH_3$ | $-(CH_2)_3-CH_3$ | H | 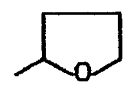 x 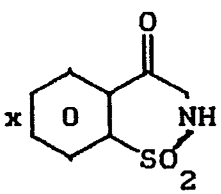 | $^1$H-NMR*): 0,8; 7,6; 7,8 |
| 61 | $(CH_3)_3C$ 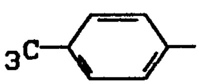 | $CH_3$ | $-(CH_2)_2-CH_3$ | H | 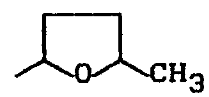 | $^1$H-NMR*): 1,3; 3,9-4,05 |
| 62 | $(CH_3)_3C$ 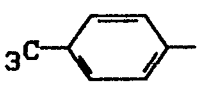 | $CH_3$ | $-(CH_2)_2-CH_3$ | H | 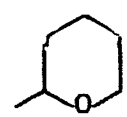 2 | $^1$H-NMR*): 1,3; 3,6-3,9 |

| Bsp. Nr. | $R^1$ | $R^2$ | $R^3$ | $R^4$ | Het | physikalische Eigenschaften |
|---|---|---|---|---|---|---|
| 63 | 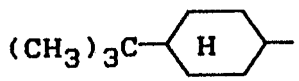 | $CH_3$ | $-(CH_2)_2-CH_3$ | H | 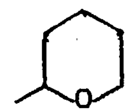 | $^1H$-NMR*): 0,8; 3,7-4,0 |
| 64 | 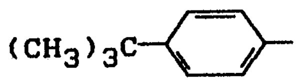 | $CH_3$ | $-(CH_2)_2-CH_3$ | H | 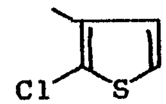 | $^1H$-NMR*): 1,3; 3,5 |
| 65 | 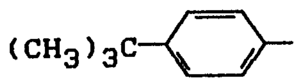 | $CH_3$ | $-(CH_2)_2-CH_3$ | H | 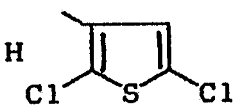 | $^1H$-NMR*): 1,3; 7,1; 7,3 |
| 66 | 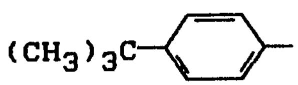 | $CH_3$ | $-(CH_2)_2-CH_3$ | H | 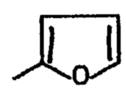 | $^1H$-NMR*): 6,1; 6,2; 6,3; 7,05; 7,25; 7,35 |

| Bsp. Nr. | $R^1$ | $R^2$ | $R^3$ | $R^4$ | Het | physikalische Eigenschaften |
|---|---|---|---|---|---|---|
| 67 | 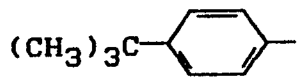 | $CH_3$ | $-(CH_2)_2-CH_3$ | H | 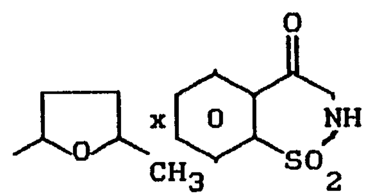 | $^1H$-NMR*): 1,3; 7,1; 7,3; 7,6; 7,8 |
| 68 | 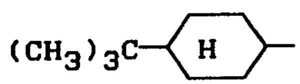 | $CH_3$ | $-(CH_2)_2-CH_3$ | H | 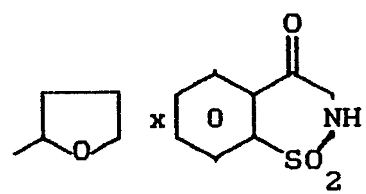 | $^1H$-NMR*): 0,8; 7,7-8,0 |
| 69 | 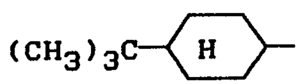 | $CH_3$ | $-(CH_2)_2-CH_3$ | H | 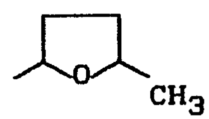 | $^1H$-NMR*): 0,8; 3,8-4,0 |
| 70 | 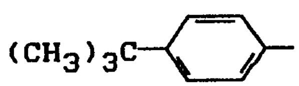 | $CH_3$ | $-(CH_2)_2-CH_3$ | H | 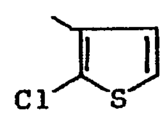 | $^1H$-NMR*): 1,3; 4,2-4,5; 7,6; 7,8 |

| Bsp. Nr. | $R^1$ | $R^2$ | $R^3$ | $R^4$ | Het | physikalische Eigenschaften |
|---|---|---|---|---|---|---|
| 71 | 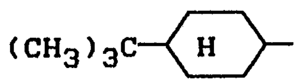 | $CH_3$ | $-(CH_2)_2-CH_3$ | H | 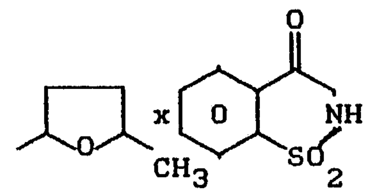 | $^1$H-NMR*): 4,0; 4,3; 7,6; 7,8; |
| 72 | 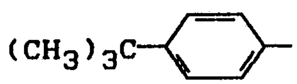 | $CH_3$ | $-(CH_2)_2-CH_3$ | H | 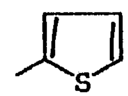 | $^1$H-NMR*): 1,3; 4,5-4,8; 7,6; 7,8 |
| 73 | 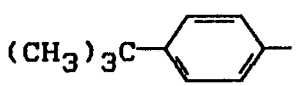 | $CH_3$ | $-(CH_2)_2-CH_3$ | H | 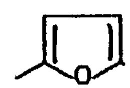 | $^1$H-NMR*): 1,3; 4,3-4,5; 7,6; 7,7-7,8 |

| Bsp. Nr. | $R^1$ | $R^2$ | $R^3$ | $R^4$ | Het | physikalische Eigenschaften |
|---|---|---|---|---|---|---|
| 74 | $(CH_3)_3C$— 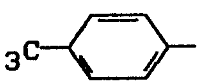 | $CH_3$ | $-(CH_2)_2-CH_3$ | H | 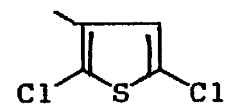 | $^1$H-NMR*) 1,3; 4,2-4,7 |
| 75 | $(CH_3)_3C$— 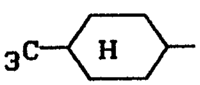 | $CH_3$ | H | H | 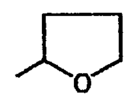 | $^1$H-NMR*) 8,8; 3,6-4,1 |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiele

In den folgenden Anwendungsbeispielen wurde die nachstehend aufgeführte Verbindung als Vergleichsubstanz eingesetzt:

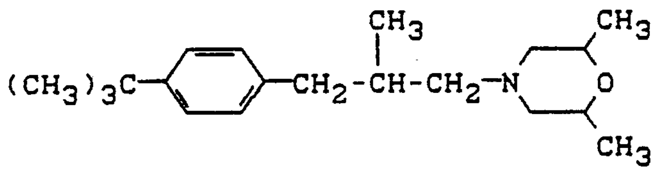 (A)

1-(4-t-Butylphenyl)-2-methyl-3-(2,6-dimethylmorpholin-4-yl)-propan
(bekannt aus DE-OS 26 56 747).

Beispiel A

Leptosphaeria nodorum-Test (Weizen)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 3 und 7.

**Ansprüche**

1. Substituierte Propylamine der allgemeinen Formel (I)

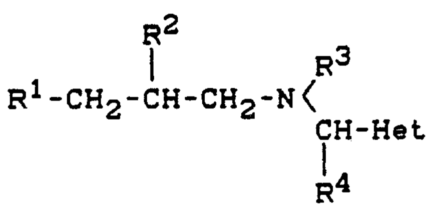 (I)

in welcher
$R^1$ für jeweils gegebenenfalls substituiertes Cycloalkyl, Cycloalkenyl, Tetrahydronaphthyl, Decahydronaphtyl, Phenyl, Naphthyl oder Thienyl steht,
$R^2$ für Alkyl steht,
$R^3$ für Wasserstoff, Alkyl, Alkenyl, Alkinyl, Hydroxyalkyl, Alkoxyalkyl, Dialkoxyalkyl, Hydroxyalkoxyalkyl, für Cyano, Formyl oder für jeweils gegebenenfalls substituiertes Cycloalkyl, Cycloalkenyl, Cycloalkylalkyl, Cycloalkenylalkyl, Aralkyl, Aryl, Furanylmethyl oder Tetrahydrofuranylmethyl steht,
$R^4$ für Wasserstoff oder Alkyl steht und
Het für einen gegebenenfalls substituierten Heterocyclus der Formel

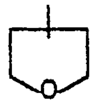 ; 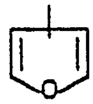 ; 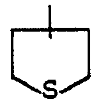 ; 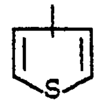 oder

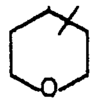

steht und deren Säureadditionssalze.

2. Substituierte Propylamine gemäß Anspruch 1, worin in der Formel (I)

$R^1$ für jeweils gegebenenfalls einfach bis mehrfach, gleich oder verschieden substituiertes Cycloalkyl oder Cycloalkenyl mit jeweils 3 bis 10 Kohlenstoffatomen steht, wobei als Substituenten infrage kommen: geradkettiges oder verzweigtes Alkyl mit 1 bis 8 Kohlenstoffatomen oder Cycloalkylalkyl mit 1 bis 8 Kohlenstoffatomen im geradkettigen oder verzweigten Alkylteil und 3 bis 7 Kohlenstoffatomen im Cycloalkylteil;

außerdem für jeweils gegebenenfalls einfach bis mehrfach, gleich oder verschieden substituiertes Tetrahydronaphthyl oder Decahydronaphthyl steht, wobei als Substituenten infrage kommen: jeweils geradkettiges oder verzweigtes Alkyl oder Alkoxy mit jeweils 1 bis 8 Kohlenstoffen;

weiterhin für jeweils gegebenenfalls einfach bis mehrfach, gleich oder verschieden substituiertes Phenyl oder Naphthyl steht, wobei als Substituenten jeweils infrage kommen: Halogen, geradkettiges oder verzweigtes Alkyl mit 1 bis 8 Kohlenstoffatomen, jeweils geradkettiges oder verzweigtes Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils gegebenenfalls durch geradkettiges oder verzweigtes Alkyl mit 1 bis 6 Kohlenstoffatomen einfach bis mehrfach, gleich oder verschieden substituiertes Cycloalkyl mit 3 bis 7 Kohlenstoffatomen, Aryl oder Aryloxy mit 6 bis 10 Kohlenstoffatomen sowie Arylalkyl mit 6 bis 10 Kohlenstoffatomen im Arylteil und 1 bis 6 Kohlenstoffatomen im geradkettigen oder verzweigten Alkylteil;

und schließlich für gegebenenfalls einfach bis mehrfach, gleich oder verschieden substituiertes Thienyl steht, wobei als Substituenten infrage kommen: Halogen, jeweils geradkettiges oder verzweigtes Alkyl oder Halogenalkyl mit jeweils 1 bis 8 Kohlenstoffatomen und gegebenenfalls 1 bis 9 gleichen oder verschiedenen Halogenatomen;

$R^2$ für geradkettiges oder verzweigtes Alkyl mit 1 bis 4 Kohlenstoffatomen steht,

$R^3$ für Wasserstoff, für geradkettiges oder verzweigtes Alkyl mit 1 bis 8 Kohlenstoffatomen, für jeweils geradkettiges oder verzweigtes Alkenyl oder Alkinyl mit jeweils 3 bis 8 Kohlenstoffatomen, für jeweils geradkettiges oder verzweigtes Alkoxyalkyl, Hydroxyalkoxy alkyl oder Dialkoxyalkyl mit jeweils 1 bis 4 Kohlenstoffatomen in den einzelnen Alkylteilen, für geradkettiges oder verzweigtes Hydroxyalkyl mit 2 bis 8 Kohlenstoffatomen, für Cyano, für Formyl, für jeweils gegebenenfalls einfach bis mehrfach, gleich oder verschieden durch geradkettiges oder verzweigtes Alkyl mit 1 bis 4 Kohlenstoffatomen substituiertes Cycloalkyl mit 3 bis 7 Kohlenstoffatomen, Cycloalkenyl mit 3 bis 7 Kohlenstoffatomen, Cycloalkylalkyl mit 3 bis 7 Kohlenstoffatomen im Cycloalkylteil und 1 bis 8 Kohlenstoffatomen im geradkettigen oder verzweigten Alkylteil, Cycloalkenylalkyl mit 3 bis 7 Kohlenstoffatomen im Cycloalkenylteil und 1 bis 8 Kohlenstoffatomen im geradkettigen oder verzweigten Alkylteil, Furanylmethyl oder Tetrahydrofuranylmethyl oder für jeweils gegebenenfalls einfach bis mehrfach, gleich oder verschieden substituiertes Phenylalkyl mit 1 bis 4 Kohlenstoffatomen im geradkettigen oder verzweigten Alkylteil oder Phenyl steht, wobei als Phenylsubstituenten jeweils infrage kommen: Halogen, jeweils geradkettiges oder verzweigtes Alkyl, Alkoxy oder Alkylthio mit jeweils 1 bis 4 Kohlenstoffatomen sowie jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen,

$R^4$ für Wasserstoff oder für geradkettiges oder verzweigtes Alkyl mit 1 bis 4 Kohlenstoffatomen steht und

Het für einen jeweils gegebenenfalls einfach bis mehrfach, gleich oder verschieden substituierten Heterocyclus der Formel

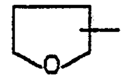 ; 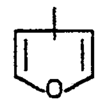 ;

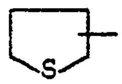 ; 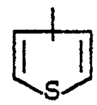 oder 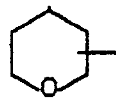

steht, wobei als Substituenten jeweils infrage kommen: jeweils geradkettiges oder verzweigtes Alkyl, Alkoxy, Alkylthio oder Alkoxycarbonyl mit jeweils 1 bis 4 Kohlenstoffatomen in den einzelnen Alkylteilen sowie jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, sowie deren Säureadditionsalze.

3. Substituierte Propylamine gemäß Anspruch 1, worin in der Formel (I)
$R^1$ für jeweils gegebenenfalls ein-bis dreifach, gleich oder verschieden substituiertes Cyclohexyl oder Cyclohexenyl steht, wobei als Substituenten jeweils infrage kommen: Methyl, Ethyl, n-oder i-Propyl, n-, i-, s-oder t-Butyl, geradkettiges oder verzweigtes Pentyl, geradkettiges oder verzweigtes Hexyl, Cyclohexylmethyl, 1-Cyclohexylethyl, 1-Cyclohexylpropyl, 1-Cyclohexylbutyl, 2-Cyclohexyl-2-propyl sowie 2-Cyclohexyl-2-butyl; außerdem für gegebenenfalls ein-bis dreifach, gleich oder verschieden substituiertes Tetrahydronaphthyl oder Decahydronaphthyl steht, wobei als Substituenten jeweils infrage kommen: Methyl, Ethyl, n-oder i-Propyl, n-, i-, s-oder t-Butyl, geradkettiges oder verzweigtes Pentyl, Methoxy, Ethoxy, n-oder i-Propoxy; weiterhin für jeweils gegebenenfalls ein-bis dreifach, gleich oder verschieden substituiertes Phenyl oder Naphthyl steht, wobei als Substituenten jeweils infrage kommen: Fluor, Chlor, Brom, Methyl, Ethyl, n-oder i-Propyl, n-, i-, s-oder t-Butyl, jeweils geradkettiges oder verzweigtes Pentyl, Hexyl, Heptyl oder Octyl, Methoxy, Ethoxy, n-oder i-Propoxy, n-, i-, s-oder t-Butoxy, Methylthio, Ethylthio, n-oder i-Propylthio, Trifluormethyl, Trifluor methoxy, Trifluormethylthio, Cyclohexyl, Phenyl, Phenoxy, Benzyl, 1-Phenylethyl, 1-Phenylpropyl, 2-Phenyl-2-propyl sowie 2-Phenyl-2-butyl, und schließlich für jeweils gegebenenfalls ein-bis dreifach, gleich oder verschieden durch Fluor, Chlor, Brom, Methyl, Ethyl, n-oder i-Propyl, n-, i-, s-oder t-Butyl oder Trifluormethyl substituiertes 2-Thienyl oder 3-Thienyl steht;
$R^2$ für Methyl, Ethyl, n-oder i-Propyl steht,
$R^3$ für Wasserstoff, Methyl, Ethyl, n-oder i-Propyl, n-, i-, s-oder t-Butyl, für jeweils geradkettiges oder verzweigtes Pentyl, Hexyl, Heptyl oder Octyl, für Allyl, Butenyl, Propargyl, Butinyl, Methoxyethyl, Methoxypropyl, Ethoxyethyl, Ethoxypropyl, Propoxypropyl, Dimethoxyethyl, Hydroxyethyl, Hydroxypropyl, Hydroxyethoxyethyl, für jeweils gegebenenfalls ein-bis dreifach, gleich oder verschieden substituiertes Cyclohexyl, Cyclohexenyl, Cyclopentyl, Cyclohexylmethyl, Cyclohexylethyl, Cyclohexenylmethyl, Cyclopentylmethyl, Furanylmethyl oder Tetrahydrofuranylmethyl steht, wobei als Substituenten jeweils infrage kommen: Methyl, Ethyl, n-oder i-Propyl, n-, i-, s-oder t-Butyl und außerdem für jeweils gegebenenfalls ein-bis dreifach gleich oder verschieden substituiertes Benzyl, Phenylethyl oder Phenyl steht, wobei als Phenylsubstituenten jeweils infrage kommen: Fluor, Chlor, Brom, Methyl, Ethyl, n-oder i-Propyl, Methoxy, Ethoxy, Methylthio, Trifluormethyl, Trifluormethoxy oder Trifluormethylthio,
$R^4$ für Wasserstoff, Methyl, Ethyl, n-oder i-Propyl steht und
Het für einen jeweils gegebenenfalls ein-bis dreifach, gleich oder verschieden substituierten Heterocyclus der Formel

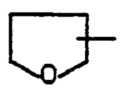 ; 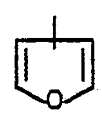 ; 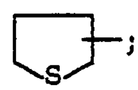 ;

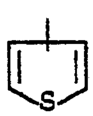 oder 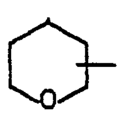

steht, wobei als Substituenten infrage kommen: Methyl, Ethyl, n-oder i-Propyl, n-, i-, s-oder t-Butyl, Methoxy, Ethoxy, n-oder i-Propoxy oder Methylthio.

4. Substituierte Propylamine gemäß Anspruch 1, worin in der Formel (I)
$R^1$ für jeweils gegebenenfalls ein-oder zweifach, gleich oder verschieden substituiertes Cyclohexyl oder Cyclohexenyl steht, wobei als Substituenten infrage kommen: i-Propyl, t-Butyl, neo-Pentyl, 1,1-Dimethylpropyl, 1,1,2-Trimethylpropyl, Cyclohexylmethyl oder 2-Cyclohexyl-2-propyl;
außerdem für gegebenenfalls ein-oder zweifach, gleich oder verschieden durch Methyl, Ethyl, Methoxy oder Ethoxy substituiertes Decahydronaphthyl steht;
weiterhin für jeweils gegebenenfalls ein-oder zweifach, gleich oder verschieden substituiertes Phenyl oder Naphthyl steht, wobei als Phenyl-Substituenten besonders bevorzugt sind: Fluor, Chlor, Brom, Isopropyl, t-Butyl, neo-Pentyl, 1,1-Dimethylpropyl, 1,1,2-Trimethylpropyl, Trifluormethyl, Trifluormethoxy, Cyclohexylmethyl, Cyclohexyl, Phenyl, Phenoxy, Benzyl, 1-Phenylethyl oder 2-Phenyl-2-propyl und wobei als Naphthyl-Substituenten besonders bevorzugt sind: Methyl, Ethyl, Methoxy oder Ethoxy und schließlich für jeweils gegebenenfalls durch Chlor, Methyl, i-Propyl oder t-Butyl substituiertes 2-Thienyl oder 3-Thienyl steht,
$R^2$ für Methyl steht,
$R^3$ für Wasserstoff, Methyl, Ethyl, n-oder i-Propyl, n-, i-oder s-Butyl, n-Pentyl, n-Hexyl, n-Octyl, Allyl, Propargyl, Methoxyethyl, Methoxypropyl, Ethoxyethyl oder Ethoxypropyl steht,
$R^4$ für Wasserstoff oder Methyl steht und
Het für einen gegebenenfalls durch Methyl substituierten Heterocyclus der Formel

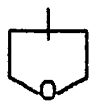 ; 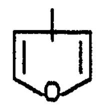 ; oder 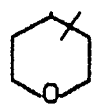

steht, sowie deren Säureadditonssalze.

5. Verfahren zur Herstellung von substituierten Propylaminen der allgemeinen Formel (I),

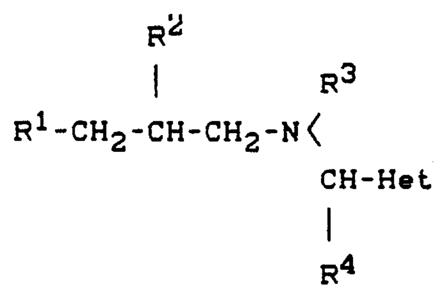 (I)

in welcher
$R^1$ für jeweils gegebenenfalls substituiertes Cycloalkyl, Cycloalkenyl, Tetrahydronaphthyl, Decahydronaphtyl, Phenyl, Naphthyl oder Thienyl steht,
$R^2$ für Alkyl steht,
$R^3$ für Wasserstoff, Alkyl, Alkenyl, Alkinyl, Hydroxyalkyl, Alkoxyalkyl, Dialkoxyalkyl, Hydroxyalkoxyalkyl, für Cyano, Formyl oder für jeweils gegebenenfalls substituiertes Cycloalkyl, Cycloalkenyl, Cycloalkylalkyl, Cycloalkenylalkyl, Aralkyl, Aryl, Furanylmethyl oder Tetrahydrofuranylmethyl steht,
$R^4$ für Wasserstoff oder Alkyl steht und
Het für einen gegebenenfalls substituierten Heterocyclus der Formel

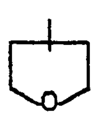 ; 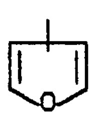 ; 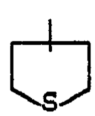 ; 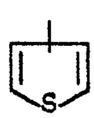 oder

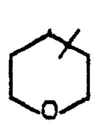

steht, sowie deren Säureadditionssalze, dadurch gekennzeichnet, daß man
(a) substituierte Aldehyde der Formel (II),

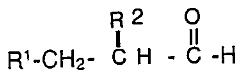 (II)

in welcher
$R^1$ und $R^2$ die oben angegebene Bedeutung haben,
mit Aminen der Formel (III),

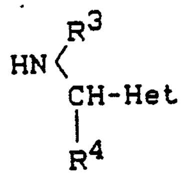 (III)

in welcher
$R^3$, $R^4$ und Het die oben angegebene Bedeutung haben,
in Gegenwart eines Reduktionsmittels sowie gegebenenfalls in Gegenwart eines Verdünnungsmittels und gegebenenfalls in Gegenwart eines Katalysators umsetzt oder daß man
(b) die mit Hilfe des erfindungsgemäßen Verfahrens (a) erhältlichen substituierten Propylamine der Formel (Ia),

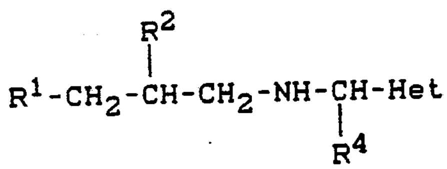 (Ia)

in welcher
$R^1$, $R^2$, $R^4$ und Het die oben angegebene Bedeutung haben,
mit Alkylierungsmitteln der Formel (IV),

$R^{3-1}$-E (IV)

in welcher
$R^{3-1}$ für Alkyl, Alkenyl, Alkinyl, Hydroxyalkyl, Alkoxyalkyl, Dialkoxyalkyl, Hydroxyalkoxyalkyl oder für jeweils gegebenenfalls substituiertes Cycloalkyl, Cycloalkenyl, Cycloalkylalkyl, Cycloalkenylalkyl, Aralkyl, Furanylmethyl oder Tetrahydrofuranylmethyl steht und
E für eine elektronenanziehende Abgangsgruppe steht,
gegebenenfalls in Gegenwart eines Verdünnungsmittels und gegebenenfalls in Gegenwart eines Reaktionshilfsmittels umsetzt, oder daß man
(c) die mit Hilfe der erfindungsgemäßen Verfahren (a) oder (b) erhältlichen substituierten Propylamine der Formel (Ib),

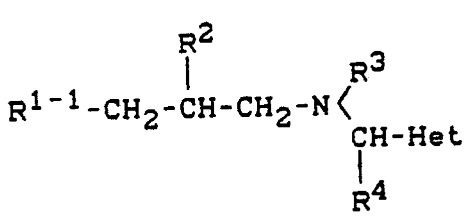 (Ib)

in welcher
$R^{1-1}$ für jeweils gegebenenfalls substituiertes Phenyl oder Naphthyl steht und
$R^2$, $R^3$, $R^4$ und Het die oben angegebene Bedeutung haben,
mit Wasserstoff in Gegenwart eines Katalysators sowie gegebenenfalls in Gegenwart eines Verdünnungsmittels hydriert und gegebenenfalls anschließend eine Säure addiert.

6. Substituierte Propylamide gemäß den Ansprüchen 1 bis 5, worin Het in der Formel (I) in 2-oder 3-Stellung verknüpft ist.

7. Schädlingsbekämpfungsmittel, gekennzeichnet durch einen Gehalt an mindestens einem substituierten Propylamin der Formel (I) nach den Ansprüchen 1 und 5.

8. Verwendung von substiuierten Propylaminen deren Formel (I) nach den Ansprüchen 1 und 5 zur Bekämpfung von Schädlingen.

9. Verfahren zur Bekämpfung von Schädlingen, dadurch gekennzeichnet, daß man substituierte Propylamine der Formel (I) nach den Ansprüchen 1 und 5 auf Schädlinge und/oder ihren Lebensraum einwirken läßt.

10. Verfahren zur Herstellung von Schädlingsbekämpfungsmitteln, dadurch gekennzeichnet, daß man substituierte Propylamine der Formel (I) nach den Ansprüchen 1 und 5 mit Streckmitteln und/oder oberflächenaktiven Mitteln vermischt.